# EUROPEAN PATENT APPLICATION

(11) **EP 3 981 879 A1**
(43) Date of publication of application: **13.04.2022**
(21) Application number: 20201096.3
(22) Date of filing: 09.10.2020
(51) Int. Cl.: C12N 15/63

(54) **METHOD OF PRODUCING A RECOMBINANT PROTEIN IN A HOST CELL WHICH HAS A DISABLED RHAMNOSE METABOLISM AS WELL AS EXPRESSION VECTORS, HOST CELLS AND RECOMBINANT PROTEINS THEREOF**

(71) Applicant: STADA ARZNEIMITTEL AG, 61118 Bad Vilbel (DE); XBRANE BIOPHARMA AB, 171 48 Solna (SE)
(72) Inventor: VIKSTRÖM, David, 194 68 Upplands Väsby (SE); ISMAIL, Nurzian, 118 27 Stockholm (SE); SAMUELSON, Patrik, 133 36 Saltsjöbaden (SE); MIRZADEH, Kiavash, 17152 SOLNA (SE); GUDISE, Santhosh, 141 56 Huddinge (SE); KADOW, Maria, 19274 Sollentuna (SE); SZEKÉR, Kathleen, 18233 Danderyd (SE); SALIH, Tagrid, 16857 Bromma Kyrka (SE); BARASZKIEWICZ, Mariusz, 16869 BROMMA (SE); BASHIRI, Siavash, 11757 Stockholm (SE)
(74) Representative: Maiwald Patent- und Rechtsanwaltsgesellschaft mbH

(57) **Abstract**

The present invention relates to DNA construct suitable for expressing a recombinant protein in a bacterial host cell wherein said DNA construct comprises nucleotide sequences encoding: a *rhaBAD* promoter, a RhaR transcription activator, a RhaS transcription activator, an antibiotic resistance marker, a promoter operably linked to the nucleic acid encoding for the antibiotic resistance marker, an rrnB T1 terminator, an rrnB T2 terminator and a pMB1 origin of replication. The DNA construct may also comprise a nucleotide sequence encoding said recombinant protein operably linked to the *rhaBAD* promoter. The present invention further relates to a vector and bacterial host cell comprising said DNA construct as well as a method of producing said recombinant protein by exposing said bacterial host cell to rhamnose and thereby inducing expression of said recombinant protein.

## Description

### TECHNICAL FIELD

The present invention relates to DNA construct suitable for expressing a recombinant protein in a bacterial host cell wherein said DNA construct comprises nucleotide sequences encoding: a *rhaBAD* promoter, a RhaR transcription activator, a RhaS transcription activator, an antibiotic resistance marker, a promoter operably linked to the nucleic acid encoding for the antibiotic resistance marker, an rrnB T1 terminator, an rrnB T2 terminator and an origin of replication. The DNA construct may also comprise a nucleotide sequence encoding said recombinant protein operably linked to the *rhaBAD* promoter. The present invention further relates to a vector and bacterial host cell comprising said DNA construct as well as a method of producing said recombinant protein by exposing said bacterial host cell to rhamnose and thereby inducing expression of said recombinant protein.

### BACKGRUND OF THE INVENTION

The metabolism of rhamnose involves L-rhamnose being taken up into cells via the permease RhaT and then isomerized into L-rhamnulose by L-rhamnose isomerase (RhaA), and L-rhamnulose is then phosphorylated further by rhamnulokinase (RhaB) and finally hydrolyzed by rhamnulose-1-phosphate aldolase (RhaD) to give dihydroxyacetonephosphate and L-lactaldehyde [1]. The genes *rhaA, rhaB* and *rhaD* form an operon referred to as *rhaBAD* and are transcribed with the aid of the *rhaBAD* promoter [1]. In comparison with other systems, the rhamnose metabolism pathway is distinguished by the fact that two transcription activators known as RhaS and RhaR are required for regulation as explained below [1].

The *rhaBAD* operon is a positively regulated catabolic operon which transcribes above mentioned *rhaB, rhaA* and *rhaD* genes divergently from the *rhaSR* operon with approximately 240 bp of DNA separating their respective transcription start sites [1]. The *rhaSR* operon encodes RhaS and RhaR wherein each monomer of the dimeric RhaS and RhaR proteins contains two helix-turn-helix motifs and contacts two major grooves of DNA. RhaR regulates transcription of *rhaSR* by binding promoter DNA spanning -32 to -82 bases relative to the *rhaSR* transcription start site [1]. Subsequent to *rhaSR* expression, RhaS bind DNA upstream of the *rhaBAD* operon at -32 to -81 bases relative to the transcription start site to increase *rhaBAD* expression [1]. Furthermore, the *rhaSR-rhaBAD* intergenic region contains CRP binding sites at positions -92.5 (CRP 1) relative to the transcription start site of the *rhaBAD* operon and CRP binding sites at positions -92.5 (CRP 2), -115.5 (CRP 3) and -116.5 (CRP 4) relative to the transcription start site of the *rhaSR* operon [1]. The cyclic AMP receptor protein (CRP) regulates the expression of more than 100 promoters in *Escherichia coli.*

DNA constructs comprising DNA sequences encoding RhaS, RhaR and the *rhaBAD* promoter are known in the art. US8138324 discloses pTACO- and pLEMO-derived plasmids (i.e. DNA constructs) comprising DNA sequences encoding RhaS, RhaR and the *rhaBAD* promoter. However, US8138324 is silent about using host cells which have a disabled rhamnose metabolism.

DNA constructs based on pRha-derived plasmids comprising DNA sequences encoding RhaS, RhaR and the *rhaBAD* promoter are also known in the art, for example from Giacalone *et al.* [5] or Hjelm *et al.* [2]. Giacalone *et al.* describe for example the plasmids pRha67A and pRha109A whereas Hjelm *et al.* disclose the plasmid pRha67K.

Although DNA constructs comprising DNA sequences encoding RhaS, RhaR and the *rhaBAD* promoter are known in the art there are still many challenges, especially in industrial scale production of recombinant proteins, in particular monoclonal antibodies or fragments thereof. The main challenges are:
(i) the host cells being subject to stress which results in damage to cellular macromolecules such as membranes, proteins and nucleic acids;
(ii) poor growth of the host cells;
(iii) poor activity of the produced recombinant proteins; and/or
(iv) obtaining recombinant proteins in low yields.

Accordingly, there is a need for improved DNA constructs as well as a host cell and method suitable for the efficient production of recombinant proteins, such as monoclonal antibodies or fragments thereof in a high yield, particularly when such recombinant proteins are intended for therapeutic applications.

### OBJECTS OF THE INVENTION

Thus, the object of the present invention was to provide improved DNA constructs, as well as a bacterial host cell and method suitable for the efficient production of recombinant proteins, such as monoclonal antibodies or fragments thereof in a bacterial host cell in a high yield, particularly when such recombinant proteins are intended for therapeutic applications.

### SUMMARY OF THE INVENTION

A first aspect of the invention relates to a DNA construct suitable for expressing a recombinant protein in a bacterial host cell, wherein said DNA construct comprises nucleotide sequences encoding:
- *rhaBAD* promoter,
- RhaR transcription activator,
- RhaS transcription activator,
- an antibiotic resistance marker,
- a promoter operably linked to the nucleotide sequence encoding the antibiotic resistance marker,
- rrnB T1 terminator,
- rrnB T2 terminator, and
- an pMB1 origin of replication.

In a preferred embodiment, the DNA construct is characterized by:
- the antibiotic resistance marker is a kanamycin resistance marker, preferably a kanamycin resistance marker comprising the nucleotide sequence of SEQ ID 12 or a sequence with at least 90 % sequence identity thereto;
- the promoter operably linked to the nucleotide sequence encoding the antibiotic resistance marker is an AmpR promoter, preferably an AmpR promoter comprising the nucleotide sequence of SEQ ID 13 or a sequence with at least 90 % sequence identity thereto;
- the rrnB T1 terminator comprising the nucleotide sequence of SEQ ID 14 or a sequence with at least 90 % sequence identity thereto;
- the rrnB T2 terminator comprising the nucleotide sequence of SEQ ID 15 or a sequence with at least 90 % sequence identity thereto; and/or
- the pMB1 origin of replication comprising the nucleotide sequence of SEQ ID 16 or a sequence with at least 90 % sequence identity thereto.

In a preferred embodiment, the DNA construct is characterized by:
- the antibiotic resistance marker being a kanamycin resistance marker comprising the nucleotide sequence of SEQ ID 12;
- the promoter operably linked to the nucleotide sequence encoding the antibiotic resistance marker is an AmpR promoter comprising the nucleotide sequence of SEQ ID 13;
- the rrnB T1 terminator comprising the nucleotide sequence of SEQ ID 14;
- the rrnB T2 terminator comprising the nucleotide sequence of SEQ ID 15; and/or
- the pMB1 origin of replication comprising the nucleotide sequence of SEQ ID 16.

In a preferred embodiment, the DNA construct is characterized by:
- the *rhaBAD* promoter comprising the nucleotide sequence of SEQ ID 8 or a sequence with at least 90 % sequence identity thereto;
- the RhaR transcription activator comprising the nucleotide sequence of SEQ ID 9 or a sequence with at least 90 % sequence identity thereto;
- the RhaS transcription activator comprising the nucleotide sequence of SEQ ID 11 or a sequence with at least 90 % sequence identity thereto;
- the antibiotic resistance marker is a kanamycin resistance marker comprising the nucleotide sequence of SEQ ID 12 or a sequence with at least 90 % sequence identity thereto;
- the AmpR promoter comprising the nucleotide sequence of SEQ ID 13 or a sequence with at least 90 % sequence identity thereto;
- the rrnB T1 terminator comprising the nucleotide sequence of SEQ ID 14 or a sequence with at least 90 % sequence identity thereto;
- the rrnB T2 terminator comprising the nucleotide sequence of SEQ ID 15 or a sequence with at least 90 % sequence identity thereto; and
- the pMB1 origin of replication comprising the nucleotide sequence of SEQ ID 16 or a sequence with at least 90 % sequence identity thereto.

In a preferred embodiment, the DNA construct is characterized by:
- the *rhaBAD* promoter comprising the nucleotide sequence of SEQ ID 8;
- the RhaR transcription activator comprising the nucleotide sequence of SEQ ID 9;
- the RhaS transcription activator comprising the nucleotide sequence of SEQ ID 11;
- the antibiotic resistance marker is a kanamycin resistance marker comprising the nucleotide sequence of SEQ ID 12;
- the AmpR promoter comprising the nucleotide sequence of SEQ ID 13;
- the rrnB T1 terminator comprising the nucleotide sequence of SEQ ID 14;
- the rrnB T2 terminator comprising the nucleotide sequence of SEQ ID 15; and
- the pMB1 origin of replication comprising the nucleotide sequence of SEQ ID 16.

In a preferred embodiment, the DNA construct comprises a multiple cloning site comprising 17 restriction sites cleavable by restriction enzymes EcoRI, NdeI, NotI, XhoI, PspXI, PaeR71, BbsI, StyI, AvrII, BanI, Acc65I, KpnI, Eco53kI, SacI, BamHI, XbaI, SalI, AccI, PstI, Sbfl, SphI and HindIII.

In a preferred embodiment, the DNA construct comprises the nucleotide sequence of SEQ ID 1, or a sequence with at least 90 % sequence identity thereto, preferably the nucleotide sequence of SEQ ID 1.

In a preferred embodiment, the DNA construct further comprises a nucleotide sequence encoding said recombinant protein operably linked to the *rhaBAD* promoter, wherein said recombinant protein is a monoclonal antibody or fragment thereof, preferably said recombinant protein is ranibizumab or a fragment thereof; and more preferably said recombinant protein is ranibizumab comprising (i) a light chain comprising the amino acid sequence of SEQ ID 3, and/or (ii) a heavy chain comprising the amino acid sequence of SEQ ID 4.

In a preferred embodiment, the DNA construct comprises a nucleotide sequence encoding the recombinant protein operably linked to the *rhaBAD* promoter comprising (i) a nucleotide sequence encoding for the light chain of ranibizumab comprising the sequence of SEQ ID 5 or a sequence with at least 90 % sequence identity thereto, and/or (ii) a nucleotide sequence encoding for the heavy chain of ranibizumab comprising the sequence of SEQ ID 6 or a sequence with at least 90 % sequence identity thereto; preferably said nucleotide sequence encoding the recombinant protein comprises (i) a nucleotide sequence encoding for the light chain of ranibizumab comprising the sequence of SEQ ID 5, and/or (ii) a nucleotide sequence encoding for the heavy chain of ranibizumab comprising the sequence of SEQ ID 6.

In an embodiment, the DNA construct further comprises a nucleotide sequence encoding the recombinant protein operably linked to the *rhaBAD* promoter comprising at least one nucleotide sequence encoding a signal peptide which is operably linked in the direction of transcription to either one or both of the nucleotide sequence of SEQ ID 5 and SEQ ID 6, preferably the signal peptide is PelB (pectate lyase B); more preferably the nucleotide sequence encoding the PelB signal peptide comprises a sequence of SEQ ID 7 or a sequence with at least 90 % sequence identity thereto; most preferably the nucleotide sequence encoding the PelB signal peptide comprises the sequence of SEQ ID 7.

In one embodiment, the resulting PelB signal peptide comprises an amino acid sequence of SEQ ID 18 [6]: MKYLLPTAAAGLLLLAAQPAMA.

In a preferred embodiment, the DNA construct comprises the sequence of SEQ ID 17 or a sequence with at least 90 % sequence identity thereto, preferably comprises the sequence of SEQ ID 17.

A second aspect of the invention relates to an expression vector comprising any of the DNA constructs according to the first aspect of the invention.

A third aspect of the invention relates to a bacterial host cell comprising a DNA construct according to the first aspect of the invention or the expression vector according to the second aspect of the invention, wherein said bacterial host cell is preferably an *Escherichia coli* cell, more preferably an *E. coli* K-12 cell.

In a preferred embodiment, said bacterial host cell either comprises (i) a chromosome which comprises a mutation in the nucleic acid sequence of the *rha*B gene which renders RhaB inactive, or (ii) a chromosome in which the nucleotide sequence encoding RhaB is deleted.

In a preferred embodiment, said bacterial host cell is an *Escherichia coli* W3110 cell, preferably comprising a chromosome which comprises a frame shift-mutation in the nucleotide sequence encoding RhaB.

In a preferred embodiment, said bacterial host cell is an *Escherichia coli* W3110 cell comprising a chromosome which comprises nucleotide sequence of SEQ ID 2 or a sequence with at least 90 % sequence identity thereto, preferably the nucleotide sequence of SEQ ID 2, wherein optionally the chromosome of the bacterial host cell further comprises a nucleotide sequence encoding RhaT.

A fourth aspect of the invention relates to a method of producing a recombinant protein comprising the step of exposing the bacterial host cell according to third aspect of the invention to rhamnose, thereby inducing expression of said recombinant protein. In a preferred embodiment, the method further comprises the step of recovering the recombinant protein from the bacterial host cell; and optionally further comprises one or more step(s) of purifying the recovered recombinant protein, preferably by one or more chromatography steps.

A fifth aspect of the invention relates to a method of producing a recombinant protein, comprising the steps of:
cloning nucleotide sequence encoding a recombinant protein into a DNA construct which comprises a nucleotide sequence of SEQ ID 1 or a sequence with at least 90 % sequence identity thereto, preferably a nucleotide sequence of SEQ ID 1, such that the nucleotide sequence encoding a recombinant protein is operably linked to the *rhaBAD* promoter; and
a. introducing the resulting nucleotide sequence into a bacterial host cell comprising a chromosome which comprises a mutation or modification which disables rhamnose metabolism.

In a preferred embodiment, the method further comprises the step of exposing the bacterial host cell to rhamnose, thereby inducing expression of the recombinant protein.

In another embodiment, the method further comprises the step of recovering the recombinant protein from the bacterial host cell; and optionally further comprises one or more step(s) of purifying the recovered recombinant protein, preferably by one or more chromatography steps.

In a preferred embodiment, said bacterial host cell is an *Escherichia coli* cell, more preferably an *E. coli* K-12 cell, most preferably an *E. coli* W3110 cell.

In a preferred embodiment, said bacterial host cell either comprises (i) a chromosome which comprises a mutation in the nucleotide sequence of the *rhaB* gene which renders RhaB inactive, or (ii) a chromosome in which the nucleotide sequence encoding RhaB is deleted.

In a preferred embodiment, said bacterial host cell is an *E. coli* W3110 cell comprising a chromosome which comprises a frame shift-mutation in the nucleotide sequence encoding RhaB.

In a preferred embodiment, said bacterial host cell is an *E. coli* W3110 cell comprising a chromosome which comprises the nucleotide sequence of SEQ ID 2 or a sequence with at least 90 % sequence identity thereto, preferably the nucleotide sequence of SEQ ID 2, wherein optionally the chromosome of the bacterial host cell further comprises a nucleotide sequence encoding RhaT.

In a preferred embodiment, said recombinant protein to be encoded is a monoclonal antibody or fragment thereof, preferably said recombinant protein to be encoded is ranibizumab or a fragment thereof; and more preferably said recombinant protein to be encoded is ranibizumab comprising (i) a light chain comprising the amino acid sequence of SEQ ID 3, and/or (ii) a heavy chain comprising the amino acid sequence of SEQ ID .

In a preferred embodiment, said nucleotide sequence encoding a recombinant protein comprises (i) a nucleotide sequence encoding the light chain of ranibizumab comprising the sequence of SEQ ID 5 or a sequence with at least 90 % sequence identity thereto, and/or (ii) a nucleotide sequence encoding for the heavy chain of ranibizumab comprising the sequence of SEQ ID 6 or a sequence with at least 90 % sequence identity thereto; preferably said nucleotide sequence encoding the recombinant protein comprises (i) a nucleotide sequence encoding for the light chain of ranibizumab comprising the sequence of SEQ ID 5, and/or (ii) a nucleotide sequence encoding for the heavy chain of ranibizumab comprising the sequence of SEQ ID 6.

In a preferred embodiment, a nucleotide sequence comprising SEQ ID 5 and/or SEQ ID 6 further comprises a nucleotide sequence encoding a signal peptide which is operably linked in the direction of transcription to the nucleotide sequence of SEQ ID 5 and/or SEQ ID 6.

In a preferred embodiment, said nucleotide sequence encoding the signal peptide is a nucleotide sequence encoding a PelB signal peptide, preferably the nucleotide sequence encoding the PelB signal peptide comprises the sequence of SEQ ID 7 or a sequence with at least 90 % sequence identity thereto; preferably the nucleotide sequence encoding the PelB signal peptide comprises the sequence of SEQ ID 7 and is fused to the nucleotide sequence encoding the light chain and/or heavy chain of ranibizumab.

In a preferred embodiment, the resulting nucleotide sequence to be introduced into the bacterial host cell comprises the sequence of SEQ ID 17 or a sequence with at least 90 % sequence identity thereto, preferably comprises the sequence of SEQ ID 17.

A sixth aspect of the invention relates to a bacterial host cell being an *E. coli* W3110 cell and comprising a chromosome which comprises a frame shift-mutation in the nucleotide sequence encoding RhaB. In an embodiment, said *E. coli* W3110 host cell comprises a chromosome which comprises the nucleotide sequence of SEQ ID 2 or a sequence with at least 90 % sequence identity thereto, preferably the nucleotide sequence of SEQ ID 2. In a further embodiment the chromosome of the bacterial host cell comprises a nucleotide sequence encoding RhaT.

One or more of the above indicated SEQ IDs 1-18 of the various aspects of the invention (and embodiments thereof) may be replaced by a sequence with at least 90 % sequence identity thereto. The term "sequence identity" as used herein is used with regard to amino acid or nucleotide sequences and the sequence identity is over the entire length of the specified sequence. A sequence may thus be at least 90 percent, at least 92 percent, at least 95 percent, at least 96 percent, at least 97 percent, at least 98 percent or at least 99 percent, identical in sequence to the amino acid or nucleotide sequence specified. Such sequences of the invention thus include single or multiple nucleotide or amino acid alterations (additions, substitutions, insertions or deletions) to the sequences of the invention. At the amino acid level preferred sequences with the above defined sequence identity contain up to 5, e.g. only 1, 2, 3, 4 or 5, preferably 1, 2 or 3, more preferably 1 or 2, altered amino acids in the sequences of the invention.

### BRIEF DESCRIPTION OF DRAWINGS

Figure 1 - Plasmid map for KTXHIS

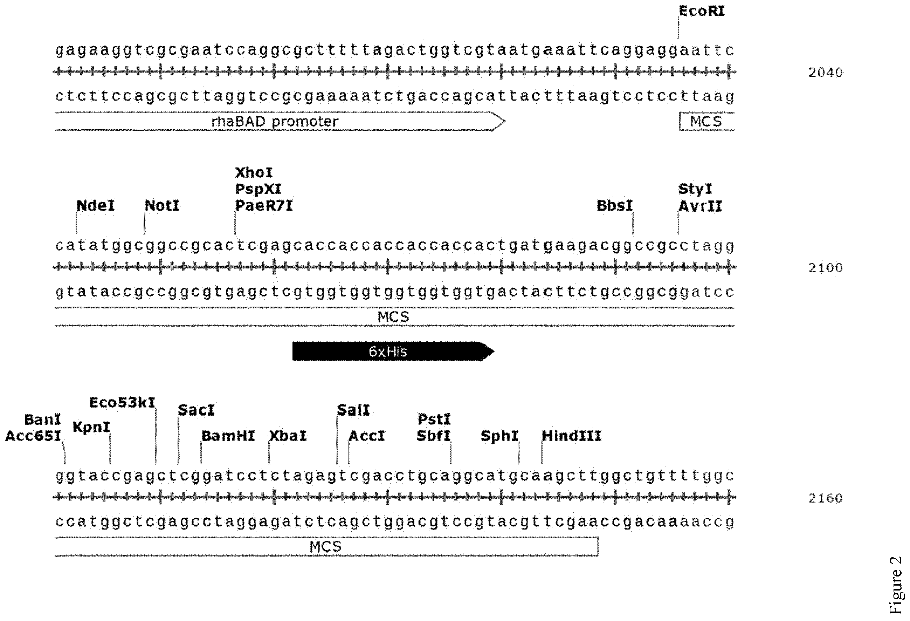

Figure 2 - The nucleotide sequence of the multiple cloning site (MCS) of KTXHIS
Figure 3 - Plasmid map of KTXHIS-PelbLC-PelbHC
Figure 4a-4d - Western blots using anti-FAB antibody - Titratability comparison in small scale experiments.
Figures 5 - Small-scale batch purification from clarified homogenized lysate samples from harvested material with Capto L resin Western blot using anti-FAB antibody. Gel loading: 3 µg product applied except for XB17/67A (only 0.2 µg product applied due to low yield). Marker from 18.4-116 kDa.
Figure 6 - Summary of Affinity HPLC titer data
Figures 7a-7c - Western blots using anti-FAB antibody - Control of expression comparison in small scale experiment.

### DETAILED DESCRIPTION

The present invention relates to regulating the L-rhamnose *rhaBAD* promoter-based production of recombinant proteins.

More specifically, the present invention relates to producing a recombinant protein comprising the steps of:
a. cloning an a nucleotide sequence encoding a recombinant protein into a DNA construct such that the nucleotide sequence is operably linked to a *rhaBAD* promoter, and
b. introducing the resulting nucleotide sequence into a bacterial host cell comprising a chromosome which comprises a mutation or modification which disables rhamnose metabolism.

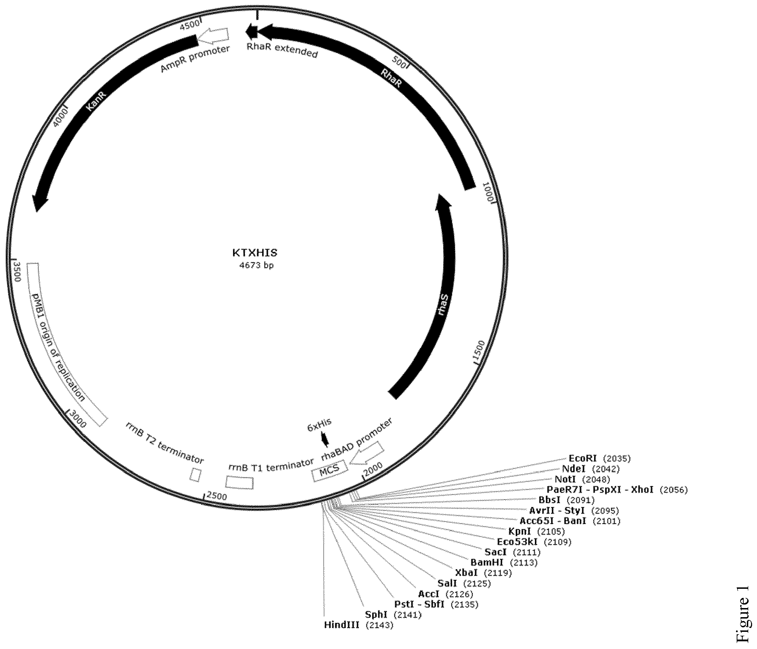

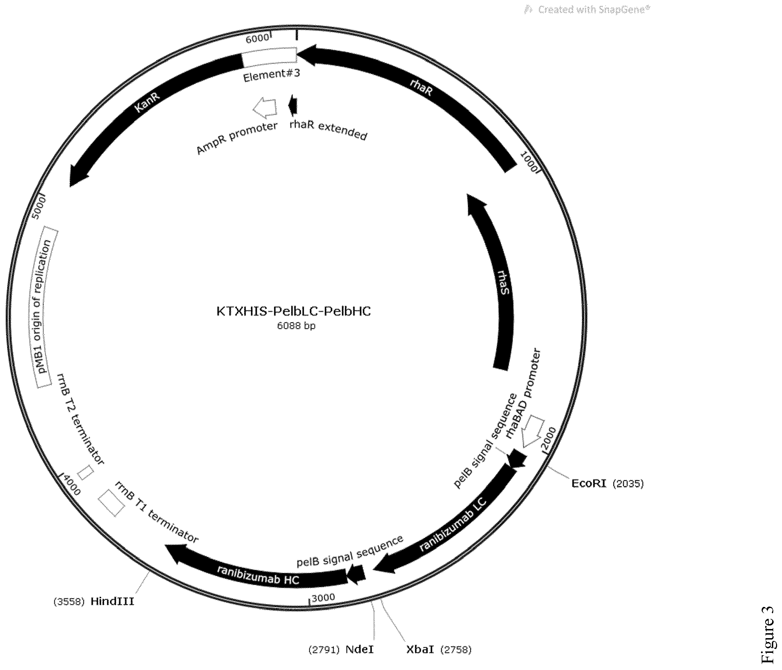

The DNA construct disclosed in the above method may comprise a nucleotide sequence encoding the *rhaBAD* promoter. In an embodiment of the invention, the nucleotide sequence of the *rhaBAD* promoter comprises the sequence of SEQ ID 8 (and wherein the sequence is referred to as "rhaBAD" in figures 1 and 3):

CACCACAATTCAGCAAATTGTGAACATCATCACGTTCATCTTTCCCTGGTTGCCAA
TGGCCCATTTTCTTGTCAGTAACGAGAAGGTCGCGAATCCAGGCGCTTTTAGAC
TGGTCGTA.

The DNA construct may comprise a nucleotide sequence encoding the RhaR transcription activator. In an embodiment of the invention, the nucleotide sequence of the RhaR transcription activator comprises a sequence of SEQ ID 9 (and wherein the sequence is referred to as "rhaR" in figures 1 and 3):

ATGGCTTTCTGCAATAACGCGAATCTTCTCAACGTATTTGTACGCCATATTGCGAA
TAATCAACTTCGTTCTCTGGCCGAGGTAGCCACGGTGGCGCATCAGTTAAAACTT
CTCAAAGATGATTTTTTGCCAGCGACCAGCAGGCAGTCGCTGTGGCTGACCGTT
ATCCGCAAGATGTCTTTGCTGAACATACACATGATTTTGTGAGCTGGTGATTGTC
TGGCGCGGTAATGGCCTGCATGTACTCAACGATCGCCCTTATCGCATTACCCGTG
GCGATCTCTTTACATTCATGCTGATGATAAACACTCCTACGCTTCCGTTAACGAT
CTGGTTTTGCAGAATATTATTTATTGCCCGGAGCGTCTGAAGCTGAATCTTGACTG
GCAGGGGGCGATTCCGGGATTTAACGCCAGCGCAGGGCAACCACACTGGCGCTT
AGGTAGCATGGGGATGGCGCAGGCGCGGCAGGTTATTGGTCAGCTTGAGCATGA
AAGTAGTCAGCATGTGCCGTTTGCTAACGAAATGGCTGAGTTGCTGTTCGGGCAG
TTGGTGATGTTGCTGAATCGCCATCGTTACACCAGTGATTCGTTGCCGCCAACATC
CAGCGAAACGTTGCTGGATAAGCTGATTACCCGGCTGGCGGCTAGCCTGAAAAGT
CCCTTTGCGCTGGATAAATTTTGTGATGAGGCATCGTGCAGTGAGCGCGTTTTGC
GTCAGCAATTTCGCCAGCAGACTGGAATGACCATCAATCAATATCTGCGACAGGT
CAGAGTGTGTCATGCGCAATATCTTCTCCAGCATAGCCGCCTGTTAATCAGTGAT
ATTTCGACCGAATGTGGCTTTGAAGATAGTAACTATTTTTCGGTGGTGTTTACCCG
GGAAACCGGGATGACGCCCAGCCAGTGGCGTCATCTCAATTCGCAGAAAGAT.

The DNA construct may further comprise a nucleotide sequence encoding an extension of the RhaR transcription activator which is in frame with RhaR because of a missing stop codon. In an embodiment of the invention, the nucleotide sequence of the extension of the RhaR transcription activator comprises the sequence of SEQ ID 10 (and wherein the sequence is referred to as "rhaR extended" in figures 1 and 3): AGACGAAAGGGCCTCGTGATACGCCTATTTTTATAG.

The DNA construct may comprise a nucleotide sequence encoding the RhaS transcription activator. In an embodiment of the invention, the nucleotide sequence of the RhaS transcription activator comprises the sequence of SEQ ID 11 (and wherein the sequence is referred to as "rhaS" in figures 1 and 3):

ATGACCGTATTACATAGTGTGGATTTTTTCCGTCTGGTAACGCGTCCGTGGCGAT
AGAACCCCGGCTCCCGCAGGCGGATTTTCCTGAACATCATCATGATTTTCATGAA
ATTGTGATTGTCGAACATGGCACGGGTATTCATGTGTTTAATGGGCAGCCCTATA
CCATCACCGGTGGCACGGTCTGTTTCGTACGCGATCATGATCGGCATCTGTATGA
ACATACCGATAATCTGTGTCTGACCAATGTGCTGTATCGCTCGCCGGATCGATTTC
AGTTTCTCGCCGGGCTGAATCAGTTGCTGCCACAAGAGCTGGATGGGCAGTATCC
GTCTCACTGGCGCGTTAACCACAGCGTATTGCAGCAGGTGCGACAGCTGGTTGCA
CAGATGGAACAGCAGGAAGGGGAAAATGATTTACCCTCGACCGCCAGTCGCGAG
ATCTTGTTTATGCAATTACTGCTCTTGCTGCGTAAAAGCAGTTTGCAGGAGAACCT
GGAAAACAGCGCATCACGTCTCAACTTGCTTCTGGCCTGGCTGGAGGACCATTTT
GCCGATGAGGTGAATTGGGATGCCGTGGCGGATCAATTTTCTCTTTCACTGCGTA
CGCTACATCGGCAGCTTAAGCAGCAAACGGGACTGACGCCTCAGCGATACCTGA
ACCGCCTGCGACTGATGAAAGCCCGACATCTGCTACGCCACAGCGAGGCCAGCG
TTACTGACATCGCCTATCGCTGTGGATTCAGCGACAGTAACCACTTTTCGACGCTT
TTTCGCCGAGAGTTTAACTGGTCACCGCGTGATATTCGCCAGGGACGGGATGGCT
TTCTGCAATAA.

The DNA construct may comprise a nucleotide sequence encoding an "antibiotic resistance marker" or "selection marker". Such a marker is a fragment of DNA that contains a gene whose product confers resistance to an antibiotic (e.g., chloramphenicol, ampicillin, gentamycin, streptomycin, tetracycline, kanamycin, neomycin) or the ability to grow on selective media (e.g., ura (uracil), leu (leucine), trp (tryptophan), his (histidine)). Usually, plasmids contain antibiotic resistance marker to force the bacterial cell to maintain the plasmid. In an embodiment of the invention, the DNA construct may comprise a nucleotide sequence of a kanamycin resistance marker. In a specific embodiment of the invention, the nucleotide sequence for conferring kanamycin resistance comprises the sequence of SEQ ID 12 (and wherein the sequence is referred to as "KanR" in figures 1 and 3):

ATGAGCCATATTCAACGGGAAACGTCTTGCTCTAGGCCGCGATTAAATTCCAACA
TGGATGCTGATTTATATGGGTATAAATGGGCTCGCGATAATGTCGGGCAATCAGG
TGCGACAATCTATCGATTGTATGGGAAGCCCGATGCGCCAGAGTTGTTTCTGAAA
CATGGCAAAGGTAGCGTTGCCAATGATGTTACAGATGAGATGGTCAGACTAAACT
GGCTGACGGAATTTATGCCTCTTCCGACCATCAAGCATTTTATCCGTACTCCTGAT
GATGCATGGTTACTCACCACTGCGATCCCCGGGAAAACAGCATTCCAGGTATTAG

AAGAATATCCTGATTCAGGTGAAAATATTGTTGATGCGCTGGCAGTGTTCCTGCG
CCGGTTGCATTCGATTCCTGTTTGTAATTGTCCTTTTAACAGCGATCGCGTATTTC
GTCTCGCTCAGGCGCAATCACGAATGAATAACGGTTTGGTTGATGCGAGTGATTT
TGATGACGAGCGTAATGGCTGGCCTGTTGAACAAGTCTGGAAAGAAATGCATAA
ACTTTTGCCATTCTCACCGGATTCAGTCGTCACTCATGGTGATTTCTCACTTGATA
ACCTTATTTTTGACGAGGGGAAATTAATAGGTTGTATTGATGTTGGACGAGTCGG
AATCGCAGACCGATACCAGGATCTTGCCATCCTATGGAACTGCCTCGGTGAGTTT
TCTCCTTCATTACAGAAACGGCTTTTTCAAAAATATGGTATTGATAATCCTGATAT
GAATAAATTGCAGTTTCATTTGATGCTCGATGAGTTTTTCTAA.

The DNA construct may comprise a nucleotide sequence encoding a promoter operably linked to the nucleic acid sequence encoding the antibiotic resistance marker. Such a promotor may increase the expression of the antibiotic resistance markers discussed in the previous paragraph. In an embodiment of the invention, the promoter for ampicillin resistance is an AmpR promoter which is not only capable of promoting expression of ampicillin resistance markers but also capable of promoting expression of kanamycin resistance markers. In a specific embodiment of the invention, the nucleic acid sequence of the AmpR promoter comprises the sequence of SEQ ID 13 (and wherein the sequence is referred to as "AmpR promoter" in figures 1 and 3):

CGCGGAACCCCTATTTGTTTATTTTTCTAAATACATTCAAATATGTATCCGCTCAT
GAGACAATAACCCTGATAAATGCTTCAATAATATTGAAAAAGGAAGAGT

The DNA construct may comprise a nucleotide sequence encoding for both of the rrnB T1 terminator and the rrnB T2 terminator. The rrnB T1 and T2 terminators are both efficient transcription terminators in isolated forms, however, when used together, rrnB T1 and T2 terminators may more efficiently terminate transcription.

In a specific embodiment of the invention, the nucleotide sequence of the rrnB T1 terminator comprises the sequence of SEQ ID 14:

CAAATAAAACGAAAGGCTCAGTCGAAAGACTGGGCCTTTCGTTTTATCTGTTGTT
TGTCGGTGAACGCTCTCCTGAGTAGGACAAAT

In a specific embodiment of the invention, the nucleotide sequence of the rrnB T2 terminator comprises the sequence of SEQ ID 15:
AGAAGGCCATCCTGACGGATGGCCTTTT

The DNA construct may further comprise an origin of replication which is a particular nucleotide sequence at which DNA replication is initiated. DNA replication may proceed from this point bidirectionally or unidirectionally. Some commonly used origins of replication are ColE1, pMB1, pSC101, R6K, pBR322, R6K, p15A, and pUC. In an embodiment of the invention, the origin of replication is pMB1 or derivatives thereof. In a specific embodiment of the invention, the nucleic acid sequence of pMB1 comprises the sequence of SEQ ID 16:

TTTCCATAGGCTCCGCCCCCCTGACGAGCATCACAAAAATCGACGCTCAAGTCAG
AGGTGGCGAAACCCGACAGGACTATAAAGATACCAGGCGTTTCCCCCTGGAAGC
TCCCTCGTGCGCTCTCCTGTTCCGACCCTGCCGCTTACCGGATACCTGTCCGCCTT
TCTCCCTTCGGGAAGCGTGGCGCTTTCTCATAGCTCACGCTGTAGGTATCTCAGTT
CGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACAC
GACTTATCGCCACTGGCAGCAGCCACTGGTAACAGGATTAGCAGAGCGAGGTAT
GTAGGCGGTGCTACAGAGTTCTTGAAGTGGTGGCCTAACTACGGCTACACTAGAA
GGACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTT
GCAAGCAGCAGATTACGCGCAGAAAAAAAGGATCTCAA

In an alternative embodiment of the invention, the DNA construct is an expression plasmid which comprises a nucleotide sequence encoding one or more of:
- *rhaBAD* promoter,
- RhaR transcription activator,
- RhaS transcription activator,
- an antibiotic resistance marker,
- a promoter operably linked to the nucleic acid encoding the antibiotic resistance marker,
- rrnB T1 terminator,
- rrnB T2 terminator, and
- an origin of replication,
wherein the DNA construct optionally comprises a multiple cloning site comprising 17 restriction sites cleavable by restriction enzymes EcoRI, NdeI, NotI, XhoI, PspXI, PaeR71, BbsI, StyI, AvrII, BanI, Acc65I, KpnI, Eco53kI, SacI, BamHI, XbaI, SalI, AccI, PstI, Sbfl, SphI and HindIII.

In an embodiment of the invention, the above disclosed expression plasmid comprises a nucleotide sequence encoding:
- *rhaBAD* promoter,
- RhaR transcription activator,
- RhaS transcription activator,
- an antibiotic resistance marker,
- a promoter operably linked to the nucleic acid sequence encoding the antibiotic resistance marker,
- rrnB T1 terminator,
- rrnB T2 terminator, and
- an origin of replication.

In an embodiment of the invention, the above disclosed expression plasmid comprises a nucleotide sequence encoding:
- *rhaBAD* promoter,
- RhaR transcription activator,
- RhaS transcription activator,
- a kanamycin resistance marker,
- AmpR promoter,
- rrnB T1 terminator,
- rrnB T2 terminator, and
- pMB1 origin of replication.

In an embodiment of the invention, the above disclosed expression plasmid comprises nucleotide sequences encoding:
- *rhaBAD* promoter comprising the nucleotide sequence of SEQ ID 8,
- RhaR transcription activator comprising the nucleotide sequence of SEQ ID 9,
- RhaS transcription activator comprising the nucleotide sequence of SEQ ID 11,
- a kanamycin resistance marker comprising the nucleotide sequence of SEQ ID 12,
- AmpR promoter comprising the nucleotide sequence of SEQ ID 13,
- rrnB T1 terminator comprising the nucleotide sequence of SEQ ID 14,
- rrnB T2 terminator comprising the nucleotide sequence of SEQ ID 15, and
- pMB1 origin of replication comprising the nucleotide sequence of SEQ ID 16.

In an embodiment of the invention, the DNA construct is a plasmid which comprises the nucleotide sequence of SEQ ID 1 and wherein said DNA construct is also referred to as KTXHIS in the present invention.

The nucleotide sequence encoding a recombinant protein which is to be cloned into the DNA constructs described above may comprise a nucleic acid encoding a monoclonal antibody or fragment thereof, preferably ranibizumab or a fragment thereof. In a specific embodiment of the invention, the nucleic acid encoding ranibizumab comprises a nucleic acid encoding the heavy and light chains of ranibizumab. Ranibizumab is a monoclonal antibody fragment (Fab) which is an anti-angiogenic that has been approved to treat the "wet" type of age-related macular degeneration, a common form of age-related vision loss.

In a specific embodiment of the invention, the nucleotide sequence encoding the heavy chain of ranibizumab comprises the sequence of SEQ ID 6:

GAAGTTCAGCTGGTTGAAAGCGGTGGTGGTCTGGTTCAGCCTGGTGGTAGCCTGC
GTCTGAGCTGTGCAGCAAGCGGTTATGATTTACCCATTATGGTATGAATTGGGTT
CGTCAGGCACCGGGTAAAGGTCTGGAATGGGTTGGTTGGATTAATACCTATACCG
GTGAACCGACCTATGCAGCAGATTTTAAACGTCGTTTTACCTTTAGCCTGGATACC
AGCAAAAGCACCGCATATCTGCAGATGAATAGCCTGCGTGCAGAGGATACCGCA
GTGTATTATTGTGCAAAATATCCGTATTATTACGGCACCAGCCATTGGTATTTCGA
TGTTTGGGGTCAGGGCACCCTGGTTACCGTTAGCAGCGCAAGCACCAAAGGTCCG
AGCGTTTTTCCGCTGGCACCGAGCAGCAAAAGTACCAGCGGTGGCACCGCAGCA
CTGGGTTGTCTGGTTAAAGATTATTTTCCGGAACCGGTTACCGTGAGCTGGAATA
GCGGTGCACTGACCAGCGGTGTTCATACCTTTCCGGCAGTTCTGCAGAGCAGCGG
TCTGTATAGCCTGAGCAGCGTTGTTACCGTTCCGAGCAGCAGCCTGGGCACCCAG

ACCTATATTTGTAATGTTAATCATAAACCGAGCAATACCAAAGTGGATAAAAAAG
TGGAACCGAAAAGCTGCGATAAAACCCATCTGTAA.

In a specific embodiment of the invention, the nucleotide sequence encoding the light chain of ranibizumab comprises the sequence of SEQ ID 5:

GATATTCAGCTGACCCAGAGCCCGAGCAGCCTGAGCGCAAGCGTTGGTGATCGTG
TTACCATTACCTGTAGCGCAAGCCAGGATATTAGCAATTATCTGAATTGGTATCA
GCAGAAACCGGGTAAAGCACCGAAAGTGCTGATCTATTTTACCAGCAGCCTGCAT
AGCGGTGTTCCGAGCCGTTTTAGCGGTAGCGGTAGTGGCACCGATTTTACCCTGA
CCATTAGCAGCCTGCAGCCGGAAGATTTTGCAACCTATTATTGTCAGCAGTATAG
CACCGTTCCGTGGACCTTTGGTCAGGGCACCAAAGTTGAAATTAAACGTACCGTT
GCAGCACCGAGCGTTTTTATCTTTCCGCCTAGTGATGAACAGCTGAAAAGCGGCA
CCGCAAGCGTTGTTTGTCTGCTGAATAACTTTTATCCGCGTGAAGCAAAAGTTCA
GTGGAAAGTTGATAATGCACTGCAGAGCGGTAATAGCCAAGAAAGCGTTACCGA
ACAGGATAGCAAAGATAGCACCTATAGCCTGAGCAGCACCCTGACCCTGAGCAA
AGCAGATTATGAAAAACACAAAGTGTATGCCTGCGAAGTTACCCATCAGGGTCTG
AGCAGTCCGGTTACCAAAAGTTTTAATCGTGGTGAATGCTAA.

In a further embodiment of the invention, the nucleic acid encoding the heavy and light chains of ranibizumab further comprises a nucleotide sequence encoding a signal peptide operably linked to either one or both of the nucleotide sequences encoding the heavy and light chains of ranibizumab. The nucleic acid sequence encoding the signal peptide may be a nucleotide sequence encoding the PelB signal peptide. In a specific embodiment of the invention, the nucleotide sequence encoding the PelB signal peptide comprises the sequence of SEQ ID 7:

ATGAAATATCTGCTGCCGACCGCAGCAGCGGGTCTGCTGCTGCTGGCAGCACAGC
CTGCAATGGCA.

The resulting nucleotide sequence (i.e. the product of cloning an nucleotide sequence encoding a recombinant protein into a DNA construct) is preferably the nucleotide sequence of SEQ ID 17 which is also referred to as KTXHIS-PelbLC-PelbHC in the present invention.

The bacterial host cell to be used for producing the recombinant protein comprises a chromosome having a mutation or modification which disables rhamnose metabolism. The bacterial host cell may be an *E. coli* cell. In a preferred embodiment, the bacterial host cell is an *E. coli* K-12 cell, more preferably the bacterial host cell is an *E. coli* W3110 cell. The disabled rhamnose metabolism is achieved by a mutation in the nucleotide sequence encoding RhaB which renders RhaB inactive. Alternatively, the disabled rhamnose metabolism is achieved by using a bacterial host cell having a chromosome in which the nucleotide sequence encoding RhaB is deleted; this can e.g. be achieved by deleting the nucleotide sequence encoding RhaB. Preferably, the chromosome of the bacterial host cell comprises the nucleic acid sequence encoding RhaT, i.e. the RhaT gene is intact.

In an embodiment of the invention, the disabled rhamnose metabolism is achieved by a frame shift-mutation in the nucleotide sequence encoding RhaB. Such a mutation results in a bacterial host cell having a chromosome which comprises the nucleotide sequence of SEQ ID 2:

GTTGGCCCCGCTCCACGCCAGTAGCGACTCGTCCCAGTCGTCGCTATTGATATTG
ACCAGTTGCGTGGTCGTGGCGTTGGTATATTCCCAGTTCATCTTGCCGGTCAGGCG
ATAACTGAAGTAATCCGGCATCAGCAGAGCGTGAGCAATGTGTGGAATAAGTTC
AGGTTGTTGCTCCGTCAGCGCACGCAACTGATAAAGCGTATTGAAGGGCAGAAA
CTGGATGCCGCTACGTTGATAAATATCGCGTTTGCCGAGTTGTTGTTGTGCCTGCG
CCATTAGGCCATTGGTGCGGCTATCGCGATAAGCAACGGGCAGGCCCACACGCTG
ACCCTGTTGGTCGAGCAGCACAAAGTCCACGCCCCAGGTATCAATCCCAATGCTA
TCGCGATACGAATCCCTTCCTCGCACACCTTGTTTAATCCAAGGCGAATGGCACTT
TCCAGGCTATCCACATCCCAGGTGACATAGCCGTTCTGACTATGCAGCCCATTGTT
AAAACGATGGATTTCGCGCAGCGTCAGGCTGCGGCATTCACGCTCGTAACGCGCC
AGCATCACGCGCCCACTGGATGCGCCGAGATCGACGGCGACACAATTGCGAAAG
GTCATAATGTGATCCTGCTGAATTTCATTACGACCAGTCTAAAAGCGCCTGAAT
TCGCGACCTTCTCGTTACTGACAGGAAAATGGGCCATTGGCAACCAGGGAAAGAT
GAACGTGATGATGTTCACAATTTGCTGAATTGTGGTGATGTGATGCTCACC.

The bacterial host cell having a chromosome which comprises the nucleotide sequence of SEQ ID 2 is in the present invention referred to as XdevK.

In one embodiment of the invention the method for developing the XdevK host cell comprises the steps of:
a) constructing the pRhaBFS plasmid, and
b) constructing of the RhaB frame shift mutant.

For constructing the pRhaBFS plasmid the two primers to PCR amplify parts of the *rhaBAD* operon from the chromosome of the *E. coli* BL21 strain (names of primers in parenthesis) are preferably:
- (SBamHI1680 - SEQ ID 19) 5' GTACGCTAGGATCCTGTGGCAGCAACTGATTC 3'
- (BSalI1681 - SEQ ID 20) 5' GTAGATAGGTCGACAGCGATCGTCATTGGGAT 3'

The present invention has multiple aspects, illustrated in examples 1-8 in the below nonlimiting EXAMPLES section. It should be understood that these examples, relating to the use of the XdevK host cell and/or the KTXHIS plasmid, while indicating preferred embodiments of the invention, are given by way of illustration only. From the above disclosed embodiments of the invention and the following examples, one skilled in the art can ascertain the essential characteristics of this invention, and without departing from the spirit and scope thereof, can make various changes and modifications of the invention to adapt it to various types of therapeutic antibodies and immunoglobulins. Thus, various modifications of the invention in addition to those shown and described herein will be apparent to those skilled in the art from the foregoing description. Such modifications are also intended to fall within the scope of the appended claims. An example of such a modification is that one or more of the above indicated SEQ IDs 1-18 may be replaced by a sequence with at least 90 % sequence identity thereto.

### EXAMPLES

Example 1 relates to the construction of the XdevK host cell.
Example 2 relates to the construction of the KTXHIS-PelbLC-PelbHC expression vector.
Example 3 relates to the light and the heavy chains of ranibizumab expressed by the expression vector in example 2.
Example 4 shows the superior titer of ranibizumab produced according to the present invention when compared to the titer of ranibizumab produced by the method described in a prior art document.
Example 5 shows that ranibizumab produced according to the present invention is free of bacteriophages.
Example 6 is a comparative experiment showing superior titratability of the present invention when compared to prior art host cells and/or plasmids.
Example 7 is a comparative experiment showing superior quality, purity and titer of the present invention when compared to prior art host cells and/or plasmids.
Example 8 is a comparative experiment showing superior control of expression of the present invention when compared to prior art host cells and/or plasmids.

### Example 1 - Strain development

The XdevK host cell is an *E. coli* W3310 derivative with a frameshift in the chromosomal copy of RhaB making it unable to utilize rhamnose as a carbon source. The XdevK host cell was developed as a strain for a rhamnose inducible system where the nucleotide sequence encoding the recombinant protein of interest is cloned into the KTXHIS plasmid and expressed under the control of a rhamnose inducible promoter.

The starting strain for the XdevK host cell was *E. coli* W3110 and a similar method of producing the XdevK host cell is described in Wilms *et al.* in the section titled "Construction of the Production Strain *E. coli* BW3110" [3].

The method for developing the XdevK host cell comprises the steps of:
c) constructing the pRhaBFS plasmid, and
d) constructing of the RhaB frame shift mutant.

### Step (a) - Construction of the pRhaBFS plasmid:

The following two primers were used to PCR amplify parts of the *rhaBAD* operon from the chromosome of the *E. coli* BL21 strain (names of primers in parenthesis):
- (SBamHI1680) 5' GTACGCTAGGATCCTGTGGCAGCAACTGATTC 3'
- (BSalI1681) 5' GTAGATAGGTCGACAGCGATCGTCATTGGGAT 3'

The PCR product was purified and digested with BamHI and SalI and ligated into a pLemo plasmid (such as pLemo described in US8138324) which had been digested with BamHI and SalI. The pLemo plasmid with the insert was then digested with ClaI. The digest was incubated with T4 polymerase to make blunt ends and then ligated. The resulting pLemo plasmid with the inserted *rhaBAD* operon genomic region with two additional bases was incubated with BamHI and SalI. The fragment containing the *rhaBAD* operon genomic region with 2 additional bases was purified and ligated with the pmak705 plasmid digested with BamHI and SalI. The plasmid resulting from the ligation was named pRhaBFS and the sequence was verified by sequencing.

### Step (b) - Construction of the RhaB frame shift mutant.

*E. coli* W3110 cells were transformed with the gene replacement plasmid pRhaBFS and then plated on LB agar vegitone containing chloramphenicol (20µg/ml) and incubated at 30 °C for 20 hours. A single colony was picked and placed in LB vegitone and grown in 30° C until the OD₆₀₀ reached 0.5. 5 µl were transferred from the culture to a LB agar vegitone plate containing chloramphenicol (20µg/ml) and incubated at 43 °C for 16 hours. Colonies were tested for correct insert into the chromosome using PCR. Colonies with correct insert were grown in LB vegitone containing chloramphenicol (20µg/ml) to saturation three times and then grow 20 hours in LB vegitone. One µl was then plated onto a LB vegitone plate. The LB vegitone plate was used as a template for replica plating onto a McConkey agar plate supplemented with 1% Rhamnose to verify that the cells cannot utilize Rhamnose as a carbon source. One colony was picked and grown in LB vegitone (Sigma) and then aliquoted and named XdevK expression strain. The chromosomal copy of the *rhaB* gene from XdevK was amplified by means of PCR. The PCR product was sequenced and the correct insertion of two bases (CG) was confirmed (see underlined CG bases in SEQ ID 2):

GTTGGCCCCGCTCCACGCCAGTAGCGACTCGTCCCAGTCGTCGCTATTGATATTG
ACCAGTTGCGTGGTCGTGGCGTTGGTATATTCCCAGTTCATCTTGCCGGTCAGGCG
ATAACTGAAGTAATCCGGCATCAGCAGAGCGTGAGCAATGTGTGGAATAAGTTC
AGGTTGTTGCTCCGTCAGCGCACGCAACTGATAAAGCGTATTGAAGGGCAGAAA
CTGGATGCCGCTACGTTGATAAATATCGCGTTTGCCGAGTTGTTGTTGTGCCTGCG

CCATTAGGCCATTGGTGCGGCTATCGCGATAAGCAACGGGCAGGCCCACACGCTG
ACCCTGTTGGTCGAGCAGCACAAAGTCCACGCCCCAGGTATCAATCCCAATGCTA
TCGCGATACGAATCCCTTCCTCGCACACCTTGTTTAATCCAAGGCGAATGGCACTT
TCCAGGCTATCCACATCCCAGGTGACATAGCCGTTCTGACTATGCAGCCCATTGTT
AAAACGATGGATTTCGCGCAGCGTCAGGCTGCGGCATTCACGCTCGTAACGCGCC
AGCATCACGCGCCCACTGGATGCGCCGAGATCGACGGCGACACAATTGCGAAAG
GTCATAATGTGATCCTGCTGAATTTCATTACGACCAGTCTAAAAAGCGCCTGAAT
TCGCGACCTTCTCGTTACTGACAGGAAAATGGGCCATTGGCAACCAGGGAAAGAT
GAACGTGATGATGTTCACAATTTGCTGAATTGTGGTGATGTGATGCTCACC

### Example 2 - KTXHIS-PelbLC-PelbHC

The PelB signal peptide comprising the nucleotide sequence of SEQ ID 7 was fused in front of nucleic acids encoding both the light and the heavy chains of ranibizumab and then cloned into the KTXHIS expression plasmid. The resulting nucleotide sequence comprises a nucleotide sequence of SEQ ID 17 and is referred to as KTXHIS-PelbLC-PelbHC in the present invention. Moreover, the plasmid map of KTXHIS-PelbLC-PelbHC is illustrated in figure 3.

The nucleotide sequence of SEQ ID 17 is disclosed below and the sequence of the nucleic acid encoding the light chain fused to PelB signal sequence is underlined and the sequence of the nucleic acid encoding the heavy chain fused to PelB signal sequence is in bold (see also figure 3 for the arrangement of PelB with respect to light and heavy chains of ranibizumab):

ATCTTTCTGCGAATTGAGATGACGCCACTGGCTGGGCGTCATCCCGGTTTCCCGG
GTAAACACCACCGAAAAATAGTTACTATCTTCAAAGCCACATTCGGTCGAAATAT
CACTGATTAACAGGCGGCTATGCTGGAGAAGATATTGCGCATGACACACTCTGAC
CTGTCGCAGATATTGATTGATGGTCATTCCAGTCTGCTGGCGAAATTGCTGACGC
AAAACGCGCTCACTGCACGATGCCTCATCACAAAATTTATCCAGCGCAAAGGGAC
TTTTCAGGCTAGCCGCCAGCCGGGTAATCAGCTTATCCAGCAACGTTTCGCTGGA
TGTTGGCGGCAACGAATCACTGGTGTAACGATGGCGATTCAGCAACATCACCAAC
TGCCCGAACAGCAACTCAGCCATTTCGTTAGCAAACGGCACATGCTGACTACTTT
CATGCTCAAGCTGACCAATAACCTGCCGCGCCTGCGCCATCCCCATGCTACCTAA
GCGCCAGTGTGGTTGCCCTGCGCTGGCGTTAAATCCCGGAATCGCCCCCTGCCAG
TCAAGATTCAGCTTCAGACGCTCCGGGCAATAAATAATATTCTGCAAAACCAGAT

CGTTAACGGAAGCGTAGGAGTGTTTATCATCAGCATGAATGTAAAAGAGATCGCC
ACGGGTAATGCGATAAGGGCGATCGTTGAGTACATGCAGGCCATTACCGCGCCA
GACAATCACCAGCTCACAAAAATCATGTGTATGTTCAGCAAAGACATCTTGCGGA
TAACGGTCAGCCACAGCGACTGCCTGCTGGTCGCTGGCAAAAAATCATCTTTGA
GAAGTTTTAACTGATGCGCCACCGTGGCTACCTCGGCCAGAGAACGAAGTTGATT
ATTCGCAATATGGCGTACAAATACGTTGAGAAGATTCGCGTTATTGCAGAAAGCC
ATCCCGTCCCTGGCGAATATCACGCGGTGACCAGTTAAACTCTCGGCGAAAAAGC
GTCGAAAAGTGGTTACTGTCGCTGAATCCACAGCGATAGGCGATGTCAGTAACGC
TGGCCTCGCTGTGGCGTAGCAGATGTCGGGCTTTCATCAGTCGCAGGCGGTTCAG
GTATCGCTGAGGCGTCAGTCCCGTTTGCTGCTTAAGCTGCCGATGTAGCGTACGC
AGTGAAAGAGAAAATTGATCCGCCACGGCATCCCAATTCACCTCATCGGCAAAAT
GGTCCTCCAGCCAGGCCAGAAGCAAGTTGAGACGTGATGCGCTGTTTTCCAGGTT
CTCCTGCAAACTGCTTTTACGCAGCAAGAGCAGTAATTGCATAAACAAGATCTCG
CGACTGGCGGTCGAGGGTAAATCATTTTCCCCTTCCTGCTGTTCCATCTGTGCAAC
CAGCTGTCGCACCTGCTGCAATACGCTGTGGTTAACGCGCCAGTGAGACGGATAC
TGCCCATCCAGCTCTTGTGGCAGCAACTGATTCAGCCCGGCGAGAAACTGAAATC
GATCCGGCGAGCGATACAGCACATTGGTCAGACACAGATTATCGGTATGTTCATA
CAGATGCCGATCATGATCGCGTACGAAACAGACCGTGCCACCGGTGATGGTATA
GGGCTGCCCATTAAACACATGAATACCCGTGCCATGTTCGACAATCACAATTTCA
TGAAAATCATGATGATGTTCAGGAAAATCCGCCTGCGGGAGCCGGGGTTCTATCG
CCACGGACGCGTTACCAGACGGAAAAAAATCCACACTATGTAATACGGTCATACT
GGCCTCCTGATGTCGTCAACACGGCGAAATAGTAATCACGAGGTCAGGTTCTTAC
CTTAAATTTCGACGGAAAACCACGTAAAAAACGTCGATTTTTCAAGATACAGCG
TGAATTTTCAGGAAATGCGGTGAGCATCACATCACCACAATTCAGCAAATTGTGA
ACATCATCACGTTCATCTTTCCCTGGTTGCCAATGGCCCATTTTCTTGTCAGTAAC
GAGAAGGTCGCGAATCCAGGCGCTTTTTAGACTGGTCGTAATGAAATTCAGGAGG
AATTCCTAATGAAATATCTGCTGCCGACCGCAGCAGCGGGTCTGCTGCTGCTGGC
AGCACAGCCTGCAATGGCAGATATTCAGCTGACCCAGAGCCCGAGCAGCCTGAG
CGCAAGCGTTGGTGATCGTGTTACCATTACCTGTAGCGCAAGCCAGGATATTAGC
AATTATCTGAATTGGTATCAGCAGAAACCGGGTAAAGCACCGAAAGTGCTGATCT
ATTTTACCAGCAGCCTGCATAGCGGTGTTCCGAGCCGTTTTAGCGGTAGCGGTAG
TGGCACCGATTTTACCCTGACCATTAGCAGCCTGCAGCCGGAAGATTTTGCAACC
TATTATTGTCAGCAGTATAGCACCGTTCCGTGGACCTTTGGTCAGGGCACCAAAG
TTGAAATTAAACGTACCGTTGCAGCACCGAGCGTTTTTATCTTTCCGCCTAGTGAT

GAACAGCTGAAAAGCGGCACCGCAAGCGTTGTTGTCTGCTGAATAACTTTATC
CGCGTGAAGCAAAAGTTCAGTGGAAAGTTGATAATGCACTGCAGAGCGGTAATA
GCCAAGAAAGCGTTACCGAACAGGATAGCAAAGATAGCACCTATAGCCTGAGCA
GCACCCTGACCCTGAGCAAAGCAGATTATGAAAAACACAAAGTGTATGCCTGCG
AAGTTACCCATCAGGGTCTGAGCAGTCCGGTTACCAAAAGTTTTAATCGTGGTGA
ATGCTAATAATCTAGAACTGTTATCGATATCAGGAGGATTAGCATATGAAATATC
TGCTGCCGACCGCAGCAGCGGGTCTGCTGCTGCTGGCAGCACAGCCTGCAA
TGGCAGAAGTTCAGCTGGTTGAAAGCGGTGGTGGTCTGGTTCAGCCTGGTG
GTAGCCTGCGTCTGAGCTGTGCAGCAAGCGGTTATGATTTTACCCATTATGG
TATGAATTGGGTTCGTCAGGCACCGGGTAAAGGTCTGGAATGGGTTGGTTG
GATTAATACCTATACCGGTGAACCGACCTATGCAGCAGATTTTAAACGTCGT
TTTACCTTTAGCCTGGATACCAGCAAAAGCACCGCATATCTGCAGATGAATA
GCCTGCGTGCAGAGGATACCGCAGTGTATTATTGTGCAAAATATCCGTATTA
TTACGGCACCAGCCATTGGTATTTCGATGTTTGGGGTCAGGGCACCCTGGTT
ACCGTTAGCAGCGCAAGCACCAAAGGTCCGAGCGTTTTTCCGCTGGCACCG
AGCAGCAAAAGTACCAGCGGTGGCACCGCAGCACTGGGTTGTCTGGTTAAA
GATTATTTTCCGGAACCGGTTACCGTGAGCTGGAATAGCGGTGCACTGACCA
GCGGTGTTCATACCTTTCCGGCAGTTCTGCAGAGCAGCGGTCTGTATAGCCT
GAGCAGCGTTGTTACCGTTCCGAGCAGCAGCCTGGGCACCCAGACCTATATT
TGTAATGTTAATCATAAACCGAGCAATACCAAAGTGGATAAAAAAGTGGAAC
CGAAAAGCTGCGATAAAACCCATCTGTAATAAAAGCTTGGCTGTTTTGGCGGA
TGAGAGAAGATTTTCAGCCTGATACAGATTAAATCAGAACGCAGAAGCGGTCTG
ATAAAACAGAATTTGCCTGGCGGCAGTAGCGCGGTGGTCCCACCTGACCCCATGC
CGAACTCAGAAGTGAAACGCCGTAGCGCCGATGGTAGTGTGGGGTCTCCCCATGC
GAGAGTAGGGAACTGCCAGGCATCAAATAAAACGAAAGGCTCAGTCGAAAGACT
GGGCCTTTCGTTTTATCTGTTGTTTGTCGGTGAACGCTCTCCTGAGTAGGACAAAT
CCGCCGGGAGCGGATTTGAACGTTGCGAAGCAACGGCCCGGAGGGTGGCGGGCA
GGACGCCCGCCATAAACTGCCAGGCATCAAATTAAGCAGAAGGCCATCCTGACG
GATGGCCTTTTTGCGTTTCTACAAACTCTTTTGTTTATTTTTCTAAATACATTCAAA
TATGTATCCGCTCATGAGACTAGGCTTCCGCGCCCTCATCCGAAAGGGCGTATTC
ATATATGCGGTGTGAAATACCGCACAGATGCGTAAGGAGAAAATACCGCATCAG
GCGCTCTTCCGCTTCCTCGCTCACTGACTCGCTGCGCTCGGTCGTTCGGCTGCGGC
GAGCGGTATCAGCTCACTCAAAGGCGGTAATACGGTTATCCACAGAATCAGGGG
ATAACGCAGGAAAGAACATGTGAGCAAAAGGCCAGCAAAAGGCCAGGAACCGT

AAAAAGGCCGCGTTGCTGGCGTTTTTCCATAGGCTCCGCCCCCCTGACGAGCATC
ACAAAAATCGACGCTCAAGTCAGAGGTGGCGAAACCCGACAGGACTATAAAGAT
ACCAGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCATAG
CTCACGCTGTAGGTATCTCAGTTCGGTGTAGGTCGTTCGCTCCAAGCTGGGCTGTG
TGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAAC
AGGATTAGCAGAGCGAGGTATGTAGGCGGTGCTACAGAGTTCTTGAAGTGGTGG
CCTAACTACGGCTACACTAGAAGGACAGTATTTGGTATCTGCGCTCTGCTGAAGC
CAGTTACCTTCGGAAAAAGAGTTGGTAGCTCTTGATCCGGCAAACAAACCACCGC
TGGTAGCGGTGGTTTTTTTGTTTGCAAGCAGCAGATTACGCGCAGAAAAAAAGGA
TCTCAAGAAGATCCTTTGATCTTTTCTACGGGGTCTGACGCTCAGTGGAACGAAA
ACTCACGTTAAGGGATTTTGGTCATGAGATTATCAAAAAGGATCTTCACCTAGAT
CCTTTTAAATTAAAAATGAAGTTTTAAATCAATCTAAAGTATATATGAGTAAACT
TGGTCTGACAGTTAGAAAAACTCATCGAGCATCAAATGAAACTGCAATTTATTCA
TATCAGGATTATCAATACCATATTTTTGAAAAAGCCGTTTCTGTAATGAAGGAGA
AAACTCACCGAGGCAGTTCCATAGGATGGCAAGATCCTGGTATCGGTCTGCGATT
CCGACTCGTCCAACATCAATACAACCTATTAATTTCCCCTCGTCAAAAATAAGGT
TATCAAGTGAGAAATCACCATGAGTGACGACTGAATCCGGTGAGAATGGCAAAA
GTTTATGCATTTCTTTCCAGACTTGTTCAACAGGCCAGCCATTACGCTCGTCATCA
AAATCACTCGCATCAACCAAACCGTTATTCATTCGTGATTGCGCCTGAGCGAGAC
GAAATACGCGATCGCTGTTAAAAGGACAATTACAAACAGGAATCGAATGCAACC
GGCGCAGGAACACTGCCAGCGCATCAACAATATTTTCACCTGAATCAGGATATTC
TTCTAATACCTGGAATGCTGTTTTCCCGGGGATCGCAGTGGTGAGTAACCATGCA
TCATCAGGAGTACGGATAAAATGCTTGATGGTCGGAAGAGGCATAAATTCCGTCA
GCCAGTTTAGTCTGACCATCTCATCTGTAACATCATTGGCAACGCTACCTTTGCCA
TGTTTCAGAAACAACTCTGGCGCATCGGGCTTCCCATACAATCGATAGATTGTCG
CACCTGATTGCCCGACATTATCGCGAGCCCATTTATACCCATATAAATCAGCATC
CATGTTGGAATTTAATCGCGGCCTAGAGCAAGACGTTTCCCGTTGAATATGGCTC
ATACTCTTCCTTTTTCAATATTATTGAAGCATTTATCAGGGTTATTGTCTCATGAG
CGGATACATATTTGAATGTATTTAGAAAAATAAACAAATAGGGGTTCCGCGCACA
TTTCCCCGAAAAGTGCCACCTGACGTCTAAGAAACCATTATTATCATGACATTAA
CCTATAAAAATAGGCGTATCACGAGGCCCTTTCGTCT

### Example 3 - Recombinant protein - ranibizumab

The amino acid sequence of the light chain of ranibizumab expressed by the expression vector KTXHIS-PelbLC-PelbHC comprises the sequence of SEQ ID 3:

DIQLTQSPSSLSASVGDRVTITCSASQDISNYLNWYQQKPGKAPKVLIYFTSSLHSGVP
SRFSGSGSGTDFTLTISSLQPEDFATYYCQQYSTVPWTFGQGTKVEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC

The amino acid sequence of the heavy chain of ranibizumab expressed by the expression vector KTXHIS-PelbLC-PelbHC comprises the sequence of SEQ ID 4:

EVQLVESGGGLVQPGGSLRLSCAASGYDFTHYGMNWVRQAPGKGLEWVGWINTYT
GEPTYAADFKRRFTFSLDTSKSTAYLQMNSLRAEDTAVYYCAKYPYYYGTSHWYFD
VWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSC
DKTHL

Moreover, the resulting PelB signal peptide comprises an amino acid sequence of SEQ ID 18 [6]: MKYLLPTAAAGLLLLAAQPAMA

### Example 4 - Superior titer of ranibizumab produced according to the present invention compared to prior art titer

3 different batches comprising ranibizumab were produced according to the below described method.

Minimal media was prepared and inoculated with overnight culture of the XdevK host cell comprising the expression vector KTXHIS-PelbLC-PelbHC from a shake flask. Fermentation temperature was set to 30 °C and the cultures were grown for 22 h in batch phase. This phase was followed by a growth phase for 10 h. The temperature was adjusted to 28.5 °C and the growth rate was then decreased by controlling the glucose feed. The culture was induced with 500 µM of rhamnose injected into the fermenter. The glucose feed was also supplemented with rhamnose for continuous induction throughout the induction phase. Material was harvested after 36 h of induction. The total fermentation time was 68 h and harvested volume was 8530 ml.

After harvest, all material, media and cells, were homogenized. 5500 ml were stored in -80°C and 3000 ml were clarified and washed using TFF. The cells were washed with a volume three times the initial volume for increased release of the product. The resulting product comprising ranibizumab was purified by using capto L affinity chromatography which is also the chromatography method used in Kumar *et al. [4]* (see sections 2.4 and 3.2 in Kumar *et al*.) and then the concentration of purified ranibizumab was determined.

The first, second and third batches comprised a ranibizumab titer of 531 mg/L, 487 mg/L and 518 mg/L, respectively.

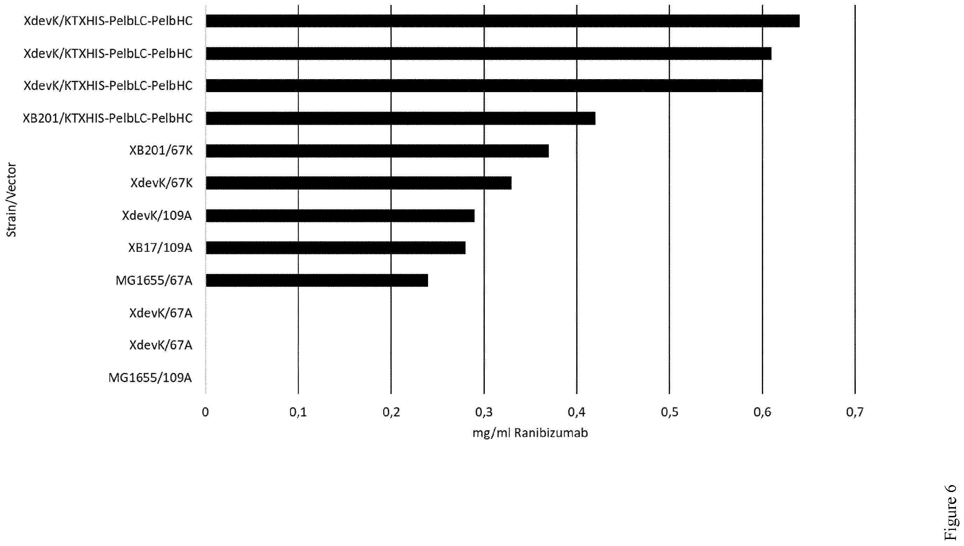

As a comparison, in prior art document Kumar *et al* (which discloses similar purification methods), the ranibizumab titer ranges from 5 mg/L to 25 mg/L as indicated in figure 6 and the Abstract of this document [4].

Consequently, the method according to the present invention (as well as the use of XdevK host cell and expression vector KTXHIS-PelbLC-PelbHC therein) provides unexpectedly higher levels of ranibizumab titer when compared to prior art methods.

### Example 5 - Cell banking

The XdevK was transformed with KTXHIS-PelbLC-PelbHC. A single colony was picked and grown in Defined Bioreactor Medium (DBM) overnight, the following day 20% glycerol was added, the culture was aliquoted and stored in -80°C. One aliquot (50 µl) was used to inoculate 100 ml of Defined Bioreactor Medium (DBM) and cultured for 24h in 30°C, temperature was shifted to 35 °C and the cells were cultured for an additional 6h to increase OD. OD A600nm = 4,44. 20% of glycerol was added, culture was aliquoted (100 µl aliquots) and frozen in -80 °C as RCB100.

Aliquots of the strain were sent for phage testing and microbial purity testing at Charles River Laboratories. The report stated: "It was concluded that RCB100 is free of bacteriophage when tested for both lysogenic prophage and free bacteriophage. The limit of the number of free bacteriophage is less than 1 plaque forming unit per mL of an overnight culture."

### Example 6 - Comparative experiments showing superior titratability

### Materials and methods

The following three strains were used in the comparative experiments:
1. XdevK host cell described in above Example 1; in other words, it is a strain which:
   - has a W3110 strain background having a genotype of: F-, λ-, IN(rrnD-rrnE)1, rph-1);
   - is a K-strain derivative and contains a mutation in the RhaB gene, making it unable to degrade Rhamnose and to use Rhamnose as a carbon source.
2. XB210 described in Hjelm *et al.* [2] which:
   - is a BL21 (DE3) strain having a genotype: F- dompt hsdSB (rB-, mB-) gal dcm (DE3));
   - is a B-strain derivative and contains a mutation in the RhaB gene and a deletion of the Rhamnose transporter gene (RhaT), making it unable to actively transport, degrade Rhamnose and to use Rhamnose as a carbon source;
   - contains parts of the DE3 prophage inserted into the chromosome and does not express the Ion protease or the OmpT protease; and
3. MG1655 described in Giacalone *et al.* [5] which is an *E*. *coli* wild type K- strain.

The following four expression plasmids were used to express the nucleic acid sequence for ranibizumab, i.e. sequences encoding the heavy and light chains of ranibizumab were cloned into the following expression plasmids:
1. KTXHIS;
2. pRha109A described in Giacalone *et al.* [5];
3. pRha67A described in Giacalone *et al.* [5]; and
4. pRha67K described in Hjelm *et al.* [2].

The resulting expression vectors were confirmed by full sequencing.

Moreover, the resulting expression vectors were introduced into the XdevK, XB201 and MG1655 strains (i.e. host cells) to test the different combinations of strains and plasmids to understand the influence on titratability, titer, quality and control of expression.

### Experimental setup for titratability comparisons

The expression vectors comprising the heavy and light chains of ranibizumab were transformed into XdevK, XB201 and MG1655 using standard protocols. Overnight cultures were prepared by inoculating a single colony in 3 ml of LB-veg liquid media containing 100 µg/ml ampicillin or 50 µg/ml kanamycin depending on the construct. Cultures were incubated in a 15 ml falcon tube at 37°C with shaking for 16 hours. Cultures were then back-diluted (1:50) into 5 ml of LB-veg plus antibiotics in a 24-well growth plate, and incubated as before until an OD600 of approximately 0.3-0.5 was reached. Expression was induced by addition of 5, 50, 100, 250, 500, 5000 µM rhamnose and incubation for 5 hours at 30 °C with shaking followed. A volume of cells corresponding to an OD600 of 0,2 units was collected, resuspended in 20 µl 2x Laemlli loading buffer, boiled for 5 mins after which 7.5 µl was analyzed by 12% mini SDS-PAGE. The gel was then immediately transferred to a nitrocellulose/PVDF membrane and probed using anti-Fab antibody for Western blot analysis.

### Results and discussion - titratability

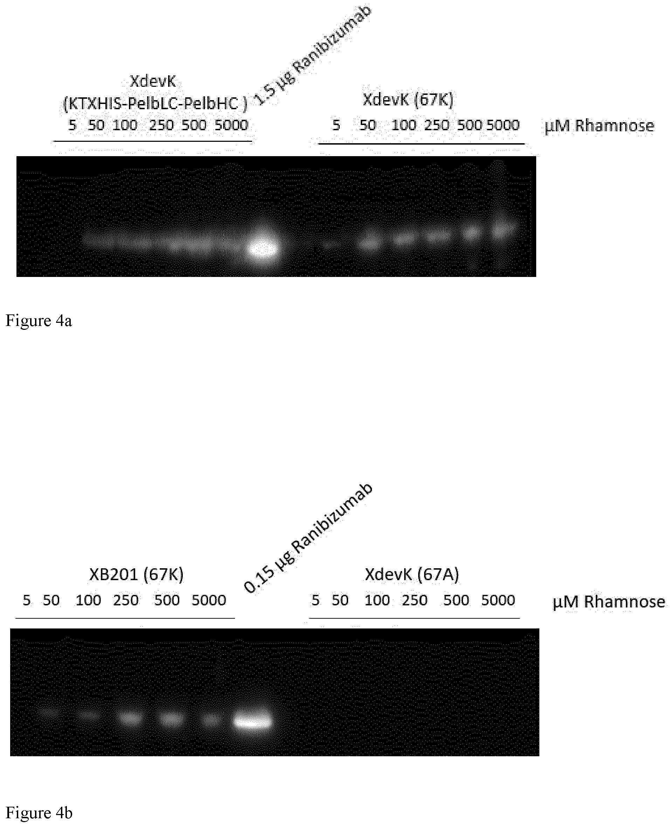

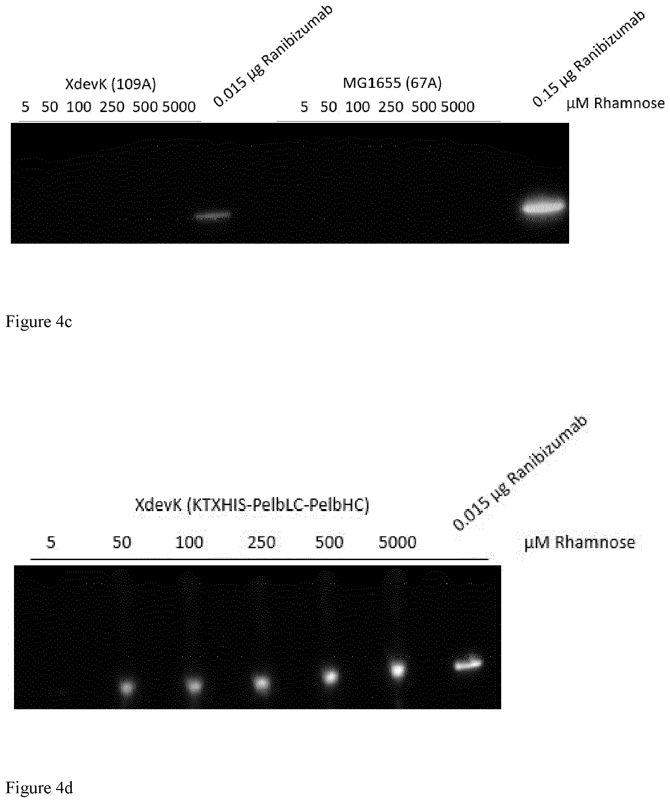

Figures 4a, 4b, 4c and 4d show the Western blots for the titratability comparisons. Each of the control lanes is loaded with purified ranibizumab.
Figure 4a shows:
   - the accumulation of expressed ranibizumab in strain XdevK in combination with KTXHIS-PelbLC-PelbHC; and
   - the accumulation of expressed ranibizumab in strain XdevK in combination with the expression vector based on the pRha67K (67K) plasmid.
Figure 4b shows:
   - the accumulation of expressed ranibizumab in strain XB201 in combination with the expression vector based on the pRha67K (67K) plasmid; and
   - the accumulation of expressed ranibizumab in strain XdevK in combination with the expression vector based on the pRha67A (67A) plasmid.
Figure 4c shows:
   - the accumulation of expressed ranibizumab in strain XdevK in combination with the expression vector based on the pRha109A (109A) plasmid; and
   - the accumulation of expressed ranibizumab in strain MG1655 in combination with the expression vector based on the pRha67A (67A) plasmid.
Figure 4d shows:
   - repeating of the experiment showing the accumulation of expressed ranibizumab in strain XdevK in combination with KTXHIS-PelbLC-PelbHC in order to visualize the titratability in combined use of XdevK and the KTXHIS-PelbLC-PelbHC setup.
Figures 4a, 4b, 4c and 4d clearly and unambiguously show that the setup with XdevK in combination with KTXHIS-PelbLC-PelbHC has superior technical effect to the other setups when comparing titratability because:
   - the expression of ranibizumab increases gradually for XdevK/KTXHIS-PelbLC-PelbHC each addition of 5, 50, 100, 250, 500, 5000 µM rhamnose; and
   - the expression of ranibizumab does not increase gradually in any other combinations - see for example the combination of XB201/67K which gives rise to a "shoulder", i.e. gradual increase but then gradual decrease.

Furthermore, figures 4a, 4b, 4c also show that the cells harboring the expression vectors based on the pRha109A or pRha67A plasmids accumulated very little ranibizumab, at least 100-fold less than the other setups using either KTXHIS-PelbLC-PelbHC or the expression vector based on the pRha67K plasmid (wherein the said fold change was observed by comparing amount of positive control loaded).

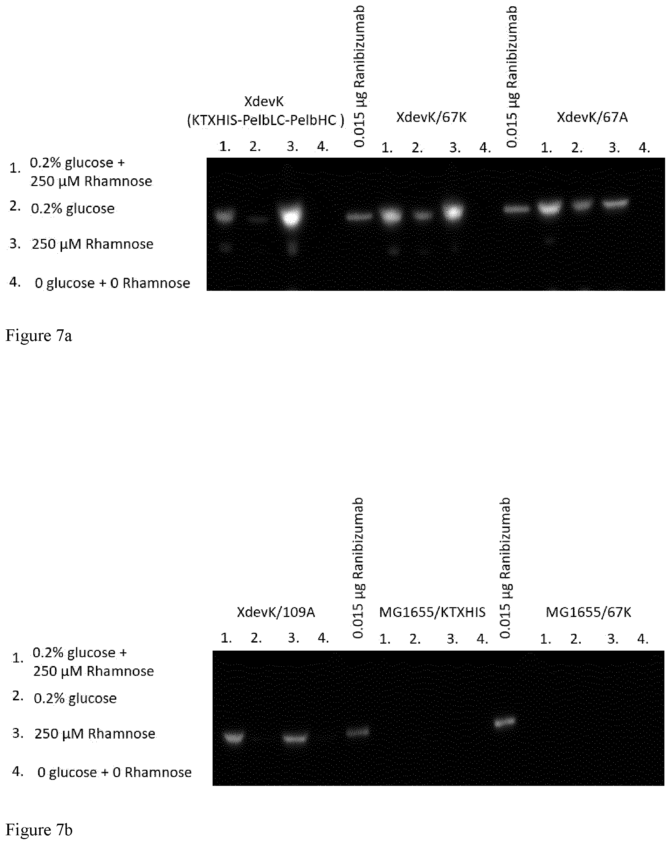

It should be noted that the combinations of:
- MG1655 and the expression vectors based on the pRha109A plasmid (see figure 6),
- MG1655 and the expression vectors based on the pRha67K plasmid (see figure 7b),
- MG1655 and KTXHIS-PelbLC-PelbHC (see figure 7b),
did not give rise to detectable production of ranibizumab and therefore titratability experiments were not conducted for said combinations.

Hence, in conclusion, the XdevK in combination with KTXHIS-PelbLC-PelbHC provides a superior titratability compared to the setups known from prior art. Thus, this allows for a precisely tuning of recombinant protein production rates and therefore the recombinant protein production rates can be set precisely and stably.

### Example 7 - Comparative experiments showing superior quality and titer

### Experimental setup for titer and quality experiments

The expression vectors comprising the heavy and light chains of ranibizumab were transformed into XdevK, XB201 and MG1655 using standard protocols to produce Research Cell Bank (RCB) of respective combination of strain and plasmid. Pre-culture (in shake flask) was inoculated from a single vial of RCB and grown overnight (O/N) in defined reactor media; Potassium dihydrogen phosphate (KH₂PO₄) 4.5 g/L, di-Potassium hydrogen phosphate (K₂HPO₄) 3.0 g/L, Ammonium sulfate ((NH₄)₂SO₄) 3.75 g/L, tri-Sodium citrate dihydrate (HOC(COONa)(CH₂COONa)₂·2H₂O) 1.88 g/L, Iron(III) chloride hexahydrate (FeCl₃·6H₂O) 0,0525 g/L, Zinc sulfate heptahydrate (ZnSO₄·7H₂O) 0.0158 g/L, Copper(II) sulfate pentahydrate (CuSO₄·5H₂O) 0.00397 g/L, Manganese sulfate monohydrate (MnSO₄·1H₂O) 0.0198 g/L, Calcium chloride dihydrate (CaCl₂·2H₂O) 0.0207 g/L add 17.1 mL sterile 60% (v/w) glucose solution, 2.432 mL 2.5 M MgSO₄·7H₂O, 1 mL 50 mg/mL Kanamycin solution. The pH was adjusted by adding 1.5 mL 25% ammonium hydroxide solution (to ca. 6.95). A DAS box fermenter was prepared with defined reactor media and inoculated with O/N pre-culture. The cells growth was controlled by feeding the culture with 60% (v/w) sterile glucose. Production phase was initiated by the addition of rhamnose as the inducer. If necessary, the feed profile was adjusted and salts were added to the culture. The cells were harvested after 20-42 h of induction followed by batch purification. The harvest samples were added to a 96-well deep-well microtiter plate that contained wells with pre-frozen water to guarantee cooling of the sample. Empty wells were filled with MilliQ water (MQ). Sonication was performed using a 24-element probe on a sonics vibra-cell device for 3 cycles of 20 sec each at an amplitude of 40 % with 30 sec pause in-between cycles. The sonicated samples were transferred to 2 mL spin tubes and centrifuged for 20 min at 20 000 x g at 4°C in a centrifuge 5430 R. The supernatant was removed carefully by pipetting and added to a 50 % slurry of the pre-equilibrated Capto L affinity resin. Equilibration had been performed three times with 20 mM sodium phosphate, 150 mM NaCl, pH 7.2 (EQ). 1.9 mL of supernatant was used to load 100 µL of Capto L slurry, which corresponds to a load rate of 9.5 mL/mL resin. The supernatant and the resin were incubated for 15 min at room temperature (RT) while shaking at 1500 rpm on a ThermoMixer C followed by centrifugation for 1 min at 2000 x g at RT. The supernatants were discarded. The resin was washed twice by adding 500 µL of EQ and separating the resin by centrifugation at 2000 x g, as above. During the third wash step using 300 µL of EQ, the resin was transferred to cellulose acetate filter spin cups suitable for 400 µL and centrifuged as above. The fourth wash step was performed with 150 µL EQ. The spin cups were transferred to new collection tubes and elution with 50 mM sodium acetate pH 3.0 was performed in three cycles using 0.4 µL of per µL resin in total (e.g. 3 x 40 µL for 50 µL slurry used). The protein concentration in the eluates was analyzed using an affinity HPLC setup. The quality of the samples was analyzed by SDS-PAGE and SCX-HPLC.

### Results and discussion for titer and quality experiments

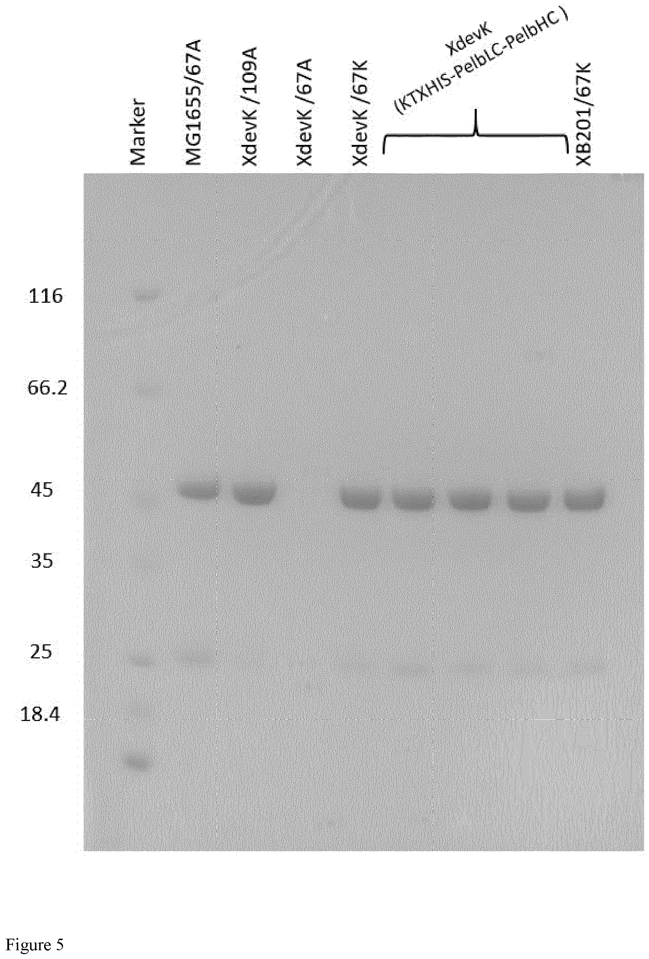

Samples were analyzed using SDS-PAGE as illustrated in figure 5. No detectable quality difference, no increased banding pattern or increased amounts of free light chain or heavy chain, could be detected. However, the setup using the XdevK strain in combination with the expression vector based on the pRha67A plasmid gave very little material and no band could be detected. Furthermore, the setup using strain XdevK in combination with the expression vector based on the pRha109A plasmid grew very poorly and was therefore harvested after a shorter induction time (of 20 hours whereas all other samples were harvested after 42 hours).

The amount of produced material was assessed using affinity HPLC and is summarized in figure 6 which clearly indicates that the setup that produced the highest yield was the XdevK strain in combination with KTXHIS-PelbLC-PelbHC.

Quality of the batch purified material was assessed with SCX-HPLC and is summarized in table 1. The material analyzed from the setup with the XdevK strain in combination with the expression vector based on the pRha67A plasmid is not reliable since this setups produces so little material that it is difficult to analyze. The material from the setup with XdevK strain in combination with the expression vector based on the pRha109A plasmid was harvested after a shorter induction time since the cells grew very slow as compared to the other cells. The SCX HPLC data summarized in table 1 clearly shows that the quality of the material produced in and purified from the XdevK strain in combination with KTXHIS-PelbLC-PelbHC is of superior purity (i.e. quality) as compared to the other setups.

**Table 1. Summary of SCX HPLC purity data.**

| **Description** | **MG1655/67A** | **XdevK /109A** | **XdevK /67A** | **XdevK /67K** | **XdevK / KTXHIS-PelbLC-PelbHC** | **XB201/67A** |
|---|---|---|---|---|---|---|
| Ranibizumab purity in % | 30.6 | 53.0 | 59.3 | 73.8 | 83.0 | 71.5 |

Hence, in conclusion, the XdevK strain in combination with KTXHIS-PelbLC-PelbHC is superior compared to the other setups both in the quality of the produced ranibizumab and the yield of the produced ranibizumab.

### Example 8 - Comparative experiments showing superior control of expression

### Experimental setup for assessing control of expression

The expression vectors comprising the heavy and light chains of ranibizumab were transformed into XdevK and MG1655 using standard protocols. Overnight cultures were prepared by inoculating a single colony in 3 ml of LB-veg liquid media containing 100 µg/ml ampicillin or 50 µg/ml kanamycin depending on the plasmid used. Cultures were incubated in a 15 ml falcon tube at 37°C with shaking for 16 hours. Cultures were then back-diluted (1:50) into 5 ml of LB-veg plus antibiotics in a 24-well growth plate, and incubated as before until an OD600 of approximately 0.3-0.5 was reached and different expression conditions were analyzed. One set of the cultures was grown in the presence of 0.2% glucose. Expression was induced by addition of 250 µM rhamnose in a subset of the cultures and incubated at 30 °C with shaking. A volume of cells corresponding to an OD600 of 0,2 units was collected (spun down) after 24 hours, resuspended in 20 µl 2x Laemlli loading buffer, boiled for 5 mins and analyzed (7,5 µl) by 12% mini SDS-PAGE. The gel was then immediately transferred to a nitrocellulose/PVDF membrane and probed using anti-Fab antibody.

### Results and discussion for control of expression

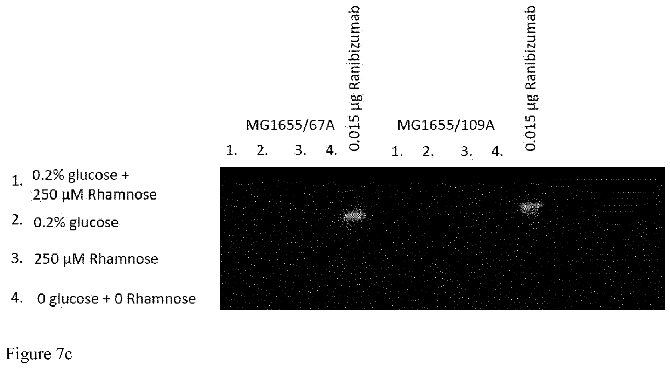

Figures 7a, 7b and 7c show the Western blots for the comparison of expression control. Each of the control lanes is loaded with ranibizumab. Samples in lane 1 from each setup were grown in the presence of 0.2% glucose and induced with 250 µm rhamnose. Samples in lane 2 from each setup were grown in the presence of 0.2% glucose. Samples in lane 3 from each setup were induced with 250 µm rhamnose. Samples in lane 4 from each setup were grown without any additions.
Figure 7a shows:
   - the accumulation of expressed ranibizumab in strain XdevK in combination with KTXHIS-PelbLC-PelbHC; and
   - the accumulation of expressed ranibizumab in strain XdevK in combination with the expression vector based on the pRha67K (67K) plasmid; and
   - the accumulation of expressed ranibizumab in strain XdevK in combination with the expression vector based on the pRha67A (67A) plasmid.
Figure 7b shows:
   - the accumulation of expressed ranibizumab in strain XdevK in combination with the expression vector based on the pRha109A (109A) plasmid;
   - the accumulation of expressed ranibizumab in strain MG1655 in combination with KTXHIS-PelbLC-PelbHC;
   - the accumulation of expressed ranibizumab in strain MG1655 in combination with the expression vector based on the pRha67K (67K) plasmid.
Figure 7c shows:
   - the accumulation of expressed ranibizumab in strain MG1655 in combination with the expression vector based on the pRha67A (67A) plasmid; and
   - the accumulation of expressed ranibizumab in strain MG1655 in combination with the expression vector based on the pRha109A (109A) plasmid.

When the accumulation of ranibizumab is compared between the different setup in figures 7a-7c, it is clear that highest expression of ranibizumab, induced by the addition of 250 µM rhamnose as indicated in lanes 3, was detected in XdevK strain in combination with KTXHIS-PelbLC-PelbHC.

Interestingly, as indicated in lanes 3 in figures 7b and 7c, none of the setups involving the MG1655 strain resulted in visible expression of ranibizumab.

Moreover, of the setups that resulted in the expression of ranibizumab in lanes 3 (i.e. the setups in which the XdevK strain was used), the XdevK strain in combination with KTXHIS-PelbLC-PelbHC resulted in the lowest expression of ranibizumab when grown in the presence of 0.2% glucose as indicated in lanes 2. This suggest that the XdevK strain in combination with KTXHIS-PelbLC-PelbHC is less prone to leaky expression in the absence of inducer. Leaky expression can have a negative effect on growth of the cells leading to lower overall yield. Leaky expression can also affect the quality of the produced material leading to a mixture of different species of the accumulated expressed protein that can be problematic to handle in a downstream purification step and therefore lead to a lower yield and a complicate purification process

Hence, the control of ranibizumab expression in the XdevK strain in combination with KTXHIS-PelbLC-PelbHC is superior when compared to the other setups.

In conclusion, the experimental results shown in Examples 4, 6, 7 and 8 demonstrate that the present invention provides the following advantageous and unexpected effects when compared with prior art expression vectors and/or host cells and therefore in particular is suitable for the industrial production of recombinant proteins
- superior titratability (example 6);
- superior purity and quality (example 7);
- superior titer (examples 4, 7 and 8); and
- superior control of expression (example 8).

### REFERENCES

1. Holcroft CC et al. Interdependence of activation at rhaSR by cyclic AMP receptor protein, the RNA polymerase alpha subunit C-terminal domain, and rhaR. J Bacteriol. 2000;182(23): 6774-6782
2. Hjelm, A. et al. Tailoring Escherichia coli for the l-Rhamnose PBAD Promoter-Based Production of Membrane and Secretory Proteins. ACS synthetic biology 2017; 6 6: 985-994.
3. Wilms, B. et al. High-cell-density fermentation for production of L-N-carbamoylase using an expression system based on the Escherichia coli rhaBAD promoter. Biotechnol. Bioeng. 2001; 73: 95-103
4. Kumar, D. et al. QbD Based Media Development for the Production of Fab Fragments in E. coli. Bioengineering 2019; 6, 29.
5. Giacalone MJ et al. "Toxic protein expression in Escherichia coli using a rhamnose-based tightly regulated and tunable promoter system. Biotechniques. 2006; 40(3): 355-364.
6. Choi, J.H. et al. Secretory and extracellular production of recombinant proteins using Escherichia coli. Appl Microbiol Biotechnol 2004; 64, 625-635

## Claims

1. DNA construct suitable for expressing a recombinant protein in a bacterial host cell, wherein said DNA construct comprises nucleotide sequences encoding:
- *rhaBAD* promoter,
- RhaR transcription activator,
- RhaS transcription activator,
- an antibiotic resistance marker,
- a promoter operably linked to the nucleotide sequence encoding the antibiotic resistance marker,
- rrnB T1 terminator,
- rrnB T2 terminator, and
- a pMB1 origin of replication.

2. DNA construct according to claim 1, wherein
- the antibiotic resistance marker is a kanamycin resistance marker, preferably a kanamycin resistance marker comprising the nucleotide sequence of SEQ ID 12 or a sequence with at least 90 % sequence identity thereto;
- the promoter operably linked to the nucleotide sequence encoding the antibiotic resistance marker is an AmpR promoter, preferably an AmpR promoter comprising the nucleotide sequence of SEQ ID 13 or a sequence with at least 90 % sequence identity thereto;
- the rrnB T1 terminator comprises a nucleotide sequence of SEQ ID 14 or a sequence with at least 90 % sequence identity thereto;
- the rrnB T2 terminator comprises the nucleotide sequence of SEQ ID 15 or a sequence with at least 90 % sequence identity thereto; and/or
- the pMB1 origin of replication comprises the nucleotide sequence of SEQ ID 16 or a sequence with at least 90 % sequence identity thereto.

3. DNA construct according to claim 1 or 2, wherein
- the antibiotic resistance marker is a kanamycin resistance marker comprising the nucleotide sequence of SEQ ID 12;
- the promoter operably linked to the nucleotide sequence encoding the antibiotic resistance marker is an AmpR promoter comprising the nucleotide sequence of SEQ ID 13;
- the rrnB T1 terminator comprises the nucleotide sequence of SEQ ID 14;
- the rrnB T2 terminator comprises the nucleotide sequence of SEQ ID 15; and/or
- the pMB1 origin of replication comprises the nucleotide sequence of SEQ ID 16.

4. DNA construct according to any one of claims 1-3, wherein:
a. the *rhaBAD* promoter comprises the nucleotide sequence of SEQ ID 8 or a sequence with at least 90 % sequence identity thereto, preferably the nucleotide sequence of SEQ ID 8;
b. the RhaR transcription activator comprises the nucleotide sequence of SEQ ID 9 or a sequence with at least 90 % sequence identity thereto, preferably the nucleotide sequence of SEQ ID 9;
c. the RhaS transcription activator comprises the nucleotide sequence of SEQ ID 11 or a sequence with at least 90 % sequence identity thereto, preferably the nucleotide sequence of SEQ ID 11;
d. the antibiotic resistance marker is a kanamycin resistance marker comprising the nucleotide sequence of SEQ ID 12 or a sequence with at least 90 % sequence identity thereto, preferably the nucleotide sequence of SEQ ID 12;
e. the AmpR promoter comprises the nucleotide sequence of SEQ ID 13 or a sequence with at least 90 % sequence identity thereto, preferably the nucleotide sequence of SEQ ID 13;
f. the rrnB T1 terminator comprises the nucleotide sequence of SEQ ID 14 or a sequence with at least 90 % sequence identity thereto, preferably the nucleotide sequence of SEQ ID 14;
g. the rrnB T2 terminator comprises the nucleotide sequence of SEQ ID 15 or a sequence with at least 90 % sequence identity thereto, preferably the nucleotide sequence of SEQ ID 15; and
h. the pMB1 origin of replication comprises the nucleotide sequence of SEQ ID 16 or a sequence with at least 90 % sequence identity thereto, preferably the nucleotide sequence of SEQ ID 16.

5. DNA construct according to any one of claims 1-4, wherein the DNA construct comprises a multiple cloning site comprising 17 restriction sites cleavable by restriction enzymes EcoRI, NdeI, NotI, XhoI, PspXI, PaeR71, BbsI, StyI, AvrII, BanI, Acc65I, KpnI, Eco53kI, SacI, BamHI, XbaI, SalI, AccI, PstI, Sbfl, SphI and HindIII.

6. DNA construct according to any one of claims 1-5, wherein said DNA construct comprises the nucleotide sequence of SEQ ID 1 or a sequence with at least 90 % sequence identity thereto, preferably the nucleotide sequence of SEQ ID 1.

7. DNA construct according to any one of 1-4, further comprising a nucleotide sequence encoding said recombinant protein, wherein the nucleotide sequence encoding the recominant protein is operably linked to the *rhaBAD* promoter, wherein said recombinant protein is a monoclonal antibody or fragment thereof; preferably said recombinant protein is ranibizumab or a fragment thereof; and more preferably said recombinant protein is ranibizumab comprising (i) a light chain comprising the amino acid sequence of SEQ ID 3 and/or (ii) a heavy chain comprising the amino acid sequence of SEQ ID 4.

8. DNA construct according to claim 7, wherein said nucleotide sequence encoding the recombinant protein comprises (i) a nucleotide sequence encoding the light chain of ranibizumab comprising the sequence of SEQ ID 5 or a sequence with at least 90 % sequence identity thereto, and/or (ii) a nucelotide sequence encoding for the heavy chain of ranibizumab comprising the sequence of SEQ ID 6 or a sequence with at least 90 % sequence identity thereto; preferably said nucleotide sequence encoding the recombinant protein comprises (i) a nucleotide sequence encoding for the light chain of ranibizumab comprising the sequence of SEQ ID 5, and/or (ii) a nucleotide sequence encoding for the heavy chain of ranibizumab comprising the sequence of SEQ ID 6.

9. DNA construct according to claim 8, wherein said nucleotide sequence encoding the recombinant protein further comprises at least one nucleotide sequence encoding a signal peptide which is operably linked in the direction of transcription to either one or both of the nucleotide sequence of SEQ ID 5 and SEQ ID 6, preferably the signal peptide is PelB; more preferably the nucleotide sequence encoding the PelB signal peptide comprises the sequence of SEQ ID 7 or a sequence with at least 90 % sequence identity thereto; most preferably the nucleotide sequence encoding the PelB signal peptide comprises the sequence of SEQ ID 7.

10. DNA construct according to claim 9, wherein said DNA construct comprises the sequence of SEQ ID 17 or a sequence with at least 90 % sequence identity thereto, preferably comprises a sequence of SEQ ID 17.

11. Expression vector comprising the DNA construct according to any one of claims 1-10.

12. Bacterial host cell comprising the DNA construct according to any of claims 1-10 or the expression vector according to claim 11, wherein said bacterial host cell is preferably an *Escherichia coli* cell, more preferably an *E. coli* K-12 cell.

13. Host cell according to claim 12, wherein said host cell either comprises (i) a chromosome which comprises a mutation in the nucleotide sequence of the *rhaB* gene which renders RhaB inactive, or (ii) a chromosome in which the nucleotide sequence encoding RhaB is deleted.

14. Host cell according to claim 12 or 13, wherein said host cell is an *Escherichia coli* W3110 cell, preferably comprising a chromosome which comprises a frame shift-mutation in the nucleotide sequence encoding RhaB.

15. Host cell according to any one of claims 12-14, wherein said host cell is an *Escherichia coli* W3110 cell comprising a chromosome which comprises the nucleotide sequence of SEQ ID 2 or a sequence with at least 90 % sequence identity thereto, preferably the nucleotide sequence of SEQ ID 2; wherein optionally, the chromosome of the host cell further comprises a nucleotide sequence encoding RhaT.

16. Method of producing a recombinant protein comprising the step of exposing the bacterial host cell according to any of claims 12-15 to rhamnose, thereby inducing expression of said recombinant protein.

17. Method according to claim 16, further comprising the step of recovering the recombinant protein from the bacterial host cell; optionally further comprising one or more step(s) of purifying the recovered recombinant protein, preferably by one or more chromatography steps.


SEQUENCE LISTING

<110> Xbrane Biopharma AB
      STADA Arzneimittel AG

<120> METHOD OF PRODUCING A RECOMBINANT PROTEIN IN A HOST CELL WHICH HAS A
DISABLED RHAMNOSE METABOLISM AS WELL AS EXPRESSION VECTORS, HOST CELLS AND
RECOMBINANT PROTEINS THEREOF

<130> S12481EP/RN

<160> 20

<170> BiSSAP 1.3.6

<210> 1
<211> 4673
<212> DNA
<213> Artificial Sequence


<220>
<223> KTXHIS

<400> 1
atctttctgc gaattgagat gacgccactg gctgggcgtc atcccggttt cccgggtaaa      60

caccaccgaa aaatagttac tatcttcaaa gccacattcg gtcgaaatat cactgattaa     120

caggcggcta tgctggagaa gatattgcgc atgacacact ctgacctgtc gcagatattg     180

attgatggtc attccagtct gctggcgaaa ttgctgacgc aaaacgcgct cactgcacga     240

tgcctcatca caaaatttat ccagcgcaaa gggacttttc aggctagccg ccagccgggt     300

aatcagctta tccagcaacg tttcgctgga tgttggcggc aacgaatcac tggtgtaacg     360

atggcgattc agcaacatca ccaactgccc gaacagcaac tcagccattt cgttagcaaa     420

cggcacatgc tgactacttt catgctcaag ctgaccaata acctgccgcg cctgcgccat     480

ccccatgcta cctaagcgcc agtgtggttg ccctgcgctg gcgttaaatc ccggaatcgc     540

cccctgccag tcaagattca gcttcagacg ctccgggcaa taaataatat tctgcaaaac     600

cagatcgtta acggaagcgt aggagtgttt atcatcagca tgaatgtaaa agagatcgcc     660

acgggtaatg cgataagggc gatcgttgag tacatgcagg ccattaccgc gccagacaat     720

caccagctca caaaaatcat gtgtatgttc agcaaagaca tcttgcggat aacggtcagc     780

cacagcgact gcctgctggt cgctggcaaa aaaatcatct ttgagaagtt ttaactgatg     840

cgccaccgtg gctacctcgg ccagagaacg aagttgatta ttcgcaatat ggcgtacaaa     900

tacgttgaga agattcgcgt tattgcagaa agccatcccg tccctggcga atatcacgcg     960

gtgaccagtt aaactctcgg cgaaaaagcg tcgaaaagtg gttactgtcg ctgaatccac    1020

agcgataggc gatgtcagta acgctggcct cgctgtggcg tagcagatgt cgggctttca    1080

tcagtcgcag gcggttcagg tatcgctgag gcgtcagtcc cgtttgctgc ttaagctgcc    1140

gatgtagcgt acgcagtgaa agagaaaatt gatccgccac ggcatcccaa ttcacctcat 1200

cggcaaaatg gtcctccagc caggccagaa gcaagttgag acgtgatgcg ctgttttcca 1260

ggttctcctg caaactgctt ttacgcagca agagcagtaa ttgcataaac aagatctcgc 1320

gactggcggt cgagggtaaa tcattttccc cttcctgctg ttccatctgt gcaaccagct 1380

gtcgcacctg ctgcaatacg ctgtggttaa cgcgccagtg agacggatac tgcccatcca 1440

gctcttgtgg cagcaactga ttcagcccgg cgagaaactg aaatcgatcc ggcgagcgat 1500

acagcacatt ggtcagacac agattatcgg tatgttcata cagatgccga tcatgatcgc 1560

gtacgaaaca gaccgtgcca ccggtgatgg tatagggctg cccattaaac acatgaatac 1620

ccgtgccatg ttcgacaatc acaatttcat gaaaatcatg atgatgttca ggaaaatccg 1680

cctgcgggag ccggggttct atcgccacgg acgcgttacc agacggaaaa aaatccacac 1740

tatgtaatac ggtcatactg gcctcctgat gtcgtcaaca cggcgaaata gtaatcacga 1800

ggtcaggttc ttaccttaaa ttttcgacgg aaaaccacgt aaaaaacgtc gatttttcaa 1860

gatacagcgt gaattttcag gaaatgcggt gagcatcaca tcaccacaat tcagcaaatt 1920

gtgaacatca tcacgttcat ctttccctgg ttgccaatgg cccattttct tgtcagtaac 1980

gagaaggtcg cgaatccagg cgctttttag actggtcgta atgaaattca ggaggaattc 2040

catatggcgg ccgcactcga gcaccaccac caccaccact gatgaagacg gccgcctagg 2100

ggtaccgagc tcggatcctc tagagtcgac ctgcaggcat gcaagcttgg ctgttttggc 2160

ggatgagaga agattttcag cctgatacag attaaatcag aacgcagaag cggtctgata 2220

aaacagaatt tgcctggcgg cagtagcgcg gtggtcccac ctgaccccat gccgaactca 2280

gaagtgaaac gccgtagcgc cgatggtagt gtggggtctc cccatgcgag agtagggaac 2340

tgccaggcat caaataaaac gaaaggctca gtcgaaagac tgggcctttc gttttatctg 2400

ttgtttgtcg gtgaacgctc tcctgagtag gacaaatccg ccgggagcgg atttgaacgt 2460

tgcgaagcaa cggcccggag ggtggcgggc aggacgcccg ccataaactg ccaggcatca 2520

aattaagcag aaggccatcc tgacggatgg cctttttgcg tttctacaaa ctcttttgtt 2580

tatttttcta aatacattca aatatgtatc cgctcatgag actaggcttc cgcgccctca 2640

tccgaaaggg cgtattcata tatgcggtgt gaaataccgc acagatgcgt aaggagaaaa 2700

taccgcatca ggcgctcttc cgcttcctcg ctcactgact cgctgcgctc ggtcgttcgg 2760

ctgcggcgag cggtatcagc tcactcaaag gcggtaatac ggttatccac agaatcaggg 2820

gataacgcag gaaagaacat gtgagcaaaa ggccagcaaa aggccaggaa ccgtaaaaag 2880

gccgcgttgc tggcgttttt ccataggctc cgcccccctg acgagcatca caaaaatcga 2940

cgctcaagtc agaggtggcg aaacccgaca ggactataaa gataccaggc gtttccccct 3000

ggaagctccc tcgtgcgctc tcctgttccg accctgccgc ttaccggata cctgtccgcc 3060

tttctccctt cgggaagcgt ggcgctttct catagctcac gctgtaggta tctcagttcg 3120

gtgtaggtcg ttcgctccaa gctgggctgt gtgcacgaac cccccgttca gcccgaccgc 3180

tgcgccttat ccggtaacta tcgtcttgag tccaacccgg taagacacga cttatcgcca 3240

ctggcagcag ccactggtaa caggattagc agagcgaggt atgtaggcgg tgctacagag 3300

ttcttgaagt ggtggcctaa ctacggctac actagaagga cagtatttgg tatctgcgct 3360

ctgctgaagc cagttacctt cggaaaaaga gttggtagct cttgatccgg caaacaaacc 3420

accgctggta gcggtggttt ttttgtttgc aagcagcaga ttacgcgcag aaaaaaagga 3480

tctcaagaag atcctttgat cttttctacg gggtctgacg ctcagtggaa cgaaaactca 3540

cgttaaggga ttttggtcat gagattatca aaaaggatct tcacctagat ccttttaaat 3600

taaaaatgaa gttttaaatc aatctaaagt atatatgagt aaacttggtc tgacagttag 3660

aaaaactcat cgagcatcaa atgaaactgc aatttattca tatcaggatt atcaatacca 3720

tatttttgaa aaagccgttt ctgtaatgaa ggagaaaact caccgaggca gttccatagg 3780

atggcaagat cctggtatcg gtctgcgatt ccgactcgtc caacatcaat acaacctatt 3840

aatttcccct cgtcaaaaat aaggttatca agtgagaaat caccatgagt gacgactgaa 3900

tccggtgaga atggcaaaag tttatgcatt tctttccaga cttgttcaac aggccagcca 3960

ttacgctcgt catcaaaatc actcgcatca accaaaccgt tattcattcg tgattgcgcc 4020

tgagcgagac gaaatacgcg atcgctgtta aaaggacaat tacaaacagg aatcgaatgc 4080

aaccggcgca ggaacactgc cagcgcatca acaatatttt cacctgaatc aggatattct 4140

tctaatacct ggaatgctgt tttcccgggg atcgcagtgg tgagtaacca tgcatcatca 4200

ggagtacgga taaaatgctt gatggtcgga agaggcataa attccgtcag ccagtttagt 4260

ctgaccatct catctgtaac atcattggca acgctacctt tgccatgttt cagaaacaac 4320

tctggcgcat cgggcttccc atacaatcga tagattgtcg cacctgattg cccgacatta 4380

tcgcgagccc atttataccc atataaatca gcatccatgt tggaatttaa tcgcggccta 4440

gagcaagacg tttcccgttg aatatggctc atactcttcc tttttcaata ttattgaagc 4500

atttatcagg gttattgtct catgagcgga tacatatttg aatgtattta gaaaaataaa 4560

caaatagggg ttccgcgcac atttccccga aaagtgccac ctgacgtcta agaaaccatt 4620

attatcatga cattaaccta taaaaatagg cgtatcacga ggccctttcg tct 4673

<210> 2
<211> 767
<212> DNA
<213> Artificial Sequence

<220>

<223> frame shift mutation

<400> 2
gttggccccg ctccacgcca gtagcgactc gtcccagtcg tcgctattga tattgaccag 60

ttgcgtggtc gtggcgttgg tatattccca gttcatcttg ccggtcaggc gataactgaa 120

gtaatccggc atcagcagag cgtgagcaat gtgtggaata agttcaggtt gttgctccgt 180

cagcgcacgc aactgataaa gcgtattgaa gggcagaaac tggatgccgc tacgttgata 240

aatatcgcgt ttgccgagtt gttgttgtgc ctgcgccatt aggccattgg tgcggctatc 300

gcgataagca acgggcaggc ccacacgctg accctgttgg tcgagcagca caaagtccac 360

gccccaggta tcaatcccaa tgctatcgcg atacgaatcc cttcctcgca caccttgttt 420

aatccaaggc gaatggcact ttccaggcta tccacatccc aggtgacata gccgttctga 480

ctatgcagcc cattgttaaa acgatggatt tcgcgcagcg tcaggctgcg gcattcacgc 540

tcgtaacgcg ccagcatcac gcgcccactg gatgcgccga gatcgacggc gacacaattg 600

cgaaaggtca taatgtgatc ctgctgaatt tcattacgac cagtctaaaa agcgcctgaa 660

ttcgcgacct tctcgttact gacaggaaaa tgggccattg gcaaccaggg aaagatgaac 720

gtgatgatgt tcacaatttg ctgaattgtg gtgatgtgat gctcacc 767

<210> 3
<211> 214
<212> PRT
<213> Artificial Sequence

<220>
<223> Ranibizumab light chain

<400> 3
Asp Ile Gln Leu Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr

180                 185                 190
Ala Cys Glu Val Thr His Gln Gly Leu Ser Ser Pro Val Thr Lys Ser
        195                 200                 205
Phe Asn Arg Gly Glu Cys
    210

<210> 4
<211> 231
<212> PRT
<213> Artificial Sequence

<220>
<223> Ranibizumab heavy chain

<400> 4
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Leu
225                 230

<210> 5
<211> 645
<212> DNA
<213> Artificial Sequence

<220>
<223> ranibizumab light chain

<400> 5
gatattcagc tgacccagag cccgagcagc ctgagcgcaa gcgttggtga tcgtgttacc      60

attacctgta gcgcaagcca ggatattagc aattatctga attggtatca gcagaaaccg     120

ggtaaagcac cgaaagtgct gatctatttt accagcagcc tgcatagcgg tgttccgagc     180

cgttttagcg gtagcggtag tggcaccgat tttaccctga ccattagcag cctgcagccg 240

gaagattttg caacctatta ttgtcagcag tatagcaccg ttccgtggac ctttggtcag 300

ggcaccaaag ttgaaattaa acgtaccgtt gcagcaccga gcgtttttat ctttccgcct 360

agtgatgaac agctgaaaag cggcaccgca agcgttgttt gtctgctgaa taacttttat 420

ccgcgtgaag caaaagttca gtggaaagtt gataatgcac tgcagagcgg taatagccaa 480

gaaagcgtta ccgaacagga tagcaaagat agcacctata gcctgagcag caccctgacc 540

ctgagcaaag cagattatga aaaacacaaa gtgtatgcct gcgaagttac ccatcagggt 600

ctgagcagtc cggttaccaa aagttttaat cgtggtgaat gctaa 645

<210> 6
<211> 696
<212> DNA
<213> Artificial Sequence

<220>
<223> ranibizumab heavy chain

<400> 6
gaagttcagc tggttgaaag cggtggtggt ctggttcagc ctggtggtag cctgcgtctg 60

agctgtgcag caagcggtta tgattttacc cattatggta tgaattgggt tcgtcaggca 120

ccgggtaaag gtctggaatg ggttggttgg attaatacct ataccggtga accgacctat 180

gcagcagatt ttaaacgtcg ttttaccttt agcctggata ccagcaaaag caccgcatat 240

ctgcagatga atagcctgcg tgcagaggat accgcagtgt attattgtgc aaaatatccg 300

tattattacg gcaccagcca ttggtatttc gatgtttggg gtcagggcac cctggttacc 360

gttagcagcg caagcaccaa aggtccgagc gtttttccgc tggcaccgag cagcaaaagt 420

accagcggtg gcaccgcagc actgggttgt ctggttaaag attattttcc ggaaccggtt 480

accgtgagct ggaatagcgg tgcactgacc agcggtgttc atacctttcc ggcagttctg 540

cagagcagcg gtctgtatag cctgagcagc gttgttaccg ttccgagcag cagcctgggc 600

acccagacct atatttgtaa tgttaatcat aaaccgagca ataccaaagt ggataaaaaa 660

gtggaaccga aaagctgcga taaaacccat ctgtaa 696

<210> 7
<211> 66
<212> DNA
<213> Artificial Sequence

<220>
<223> PelB signal peptide - DNA

<400> 7
atgaaatatc tgctgccgac cgcagcagcg ggtctgctgc tgctggcagc acagcctgca 60

atggca                                                                66

<210> 8
<211> 119
<212> DNA
<213> Artificial Sequence

<220>
<223> rhaBAD promoter

<400> 8
caccacaatt cagcaaattg tgaacatcat cacgttcatc tttccctggt tgccaatggc      60

ccattttctt gtcagtaacg agaaggtcgc gaatccaggc gctttttaga ctggtcgta      119

<210> 9
<211> 936
<212> DNA
<213> Artificial Sequence

<220>
<223> RhaR transcription activator

<400> 9
atggctttct gcaataacgc gaatcttctc aacgtatttg tacgccatat tgcgaataat      60

caacttcgtt ctctggccga ggtagccacg gtggcgcatc agttaaaact tctcaaagat     120

gatttttttg ccagcgacca gcaggcagtc gctgtggctg accgttatcc gcaagatgtc     180

tttgctgaac atacacatga tttttgtgag ctggtgattg tctggcgcgg taatggcctg     240

catgtactca acgatcgccc ttatcgcatt acccgtggcg atctctttta cattcatgct     300

gatgataaac actcctacgc ttccgttaac gatctggttt tgcagaatat tatttattgc     360

ccggagcgtc tgaagctgaa tcttgactgg cagggggcga ttccgggatt taacgccagc     420

gcagggcaac cacactggcg cttaggtagc atggggatgg cgcaggcgcg gcaggttatt     480

ggtcagcttg agcatgaaag tagtcagcat gtgccgtttg ctaacgaaat ggctgagttg     540

ctgttcgggc agttggtgat gttgctgaat cgccatcgtt acaccagtga ttcgttgccg     600

ccaacatcca gcgaaacgtt gctggataag ctgattaccc ggctggcggc tagcctgaaa     660

agtccctttg cgctggataa attttgtgat gaggcatcgt gcagtgagcg cgttttgcgt     720

cagcaatttc gccagcagac tggaatgacc atcaatcaat atctgcgaca ggtcagagtg     780

tgtcatgcgc aatatcttct ccagcatagc cgcctgttaa tcagtgatat ttcgaccgaa     840

tgtggctttg aagatagtaa ctatttttcg gtggtgttta cccgggaaac cgggatgacg     900

cccagccagt ggcgtcatct caattcgcag aaagat                               936

<210> 10

<211> 36
<212> DNA
<213> Artificial Sequence

<220>
<223> RhaR extended

<400> 10
agacgaaagg gcctcgtgat acgcctattt ttatag 36

<210> 11
<211> 837
<212> DNA
<213> Artificial Sequence

<220>
<223> RhaS transcription activator

<400> 11
atgaccgtat tacatagtgt ggattttttt ccgtctggta acgcgtccgt ggcgatagaa 60

ccccggctcc cgcaggcgga ttttcctgaa catcatcatg attttcatga aattgtgatt 120

gtcgaacatg gcacgggtat tcatgtgttt aatgggcagc cctataccat caccggtggc 180

acggtctgtt tcgtacgcga tcatgatcgg catctgtatg aacataccga taatctgtgt 240

ctgaccaatg tgctgtatcg ctcgccggat cgatttcagt ttctcgccgg gctgaatcag 300

ttgctgccac aagagctgga tgggcagtat ccgtctcact ggcgcgttaa ccacagcgta 360

ttgcagcagg tgcgacagct ggttgcacag atggaacagc aggaagggga aaatgattta 420

ccctcgaccg ccagtcgcga gatcttgttt atgcaattac tgctcttgct gcgtaaaagc 480

agtttgcagg agaacctgga aaacagcgca tcacgtctca acttgcttct ggcctggctg 540

gaggaccatt ttgccgatga ggtgaattgg gatgccgtgg cggatcaatt ttctctttca 600

ctgcgtacgc tacatcggca gcttaagcag caaacgggac tgacgcctca gcgatacctg 660

aaccgcctgc gactgatgaa agcccgacat ctgctacgcc acagcgaggc cagcgttact 720

gacatcgcct atcgctgtgg attcagcgac agtaaccact tttcgacgct ttttcgccga 780

gagtttaact ggtcaccgcg tgatattcgc cagggacggg atggctttct gcaataa 837

<210> 12
<211> 816
<212> DNA
<213> Artificial Sequence

<220>
<223> KanR

<400> 12
atgagccata ttcaacggga aacgtcttgc tctaggccgc gattaaattc caacatggat 60

gctgatttat atgggtataa atgggctcgc gataatgtcg ggcaatcagg tgcgacaatc 120

tatcgattgt atgggaagcc cgatgcgcca gagttgtttc tgaaacatgg caaaggtagc 180

gttgccaatg atgttacaga tgagatggtc agactaaact ggctgacgga atttatgcct 240

cttccgacca tcaagcattt tatccgtact cctgatgatg catggttact caccactgcg 300

atccccggga aaacagcatt ccaggtatta gaagaatatc ctgattcagg tgaaaatatt 360

gttgatgcgc tggcagtgtt cctgcgccgg ttgcattcga ttcctgtttg taattgtcct 420

tttaacagcg atcgcgtatt tcgtctcgct caggcgcaat cacgaatgaa taacggtttg 480

gttgatgcga gtgattttga tgacgagcgt aatggctggc ctgttgaaca agtctggaaa 540

gaaatgcata aacttttgcc attctcaccg gattcagtcg tcactcatgg tgatttctca 600

cttgataacc ttatttttga cgaggggaaa ttaataggtt gtattgatgt tggacgagtc 660

ggaatcgcag accgatacca ggatcttgcc atcctatgga actgcctcgg tgagttttct 720

ccttcattac agaaacggct ttttcaaaaa tatggtattg ataatcctga tatgaataaa 780

ttgcagtttc atttgatgct cgatgagttt ttctaa 816

<210> 13
<211> 105
<212> DNA
<213> Artificial Sequence

<220>
<223> AmpR promoter

<400> 13
cgcggaaccc ctatttgttt atttttctaa atacattcaa atatgtatcc gctcatgaga 60

caataaccct gataaatgct tcaataatat tgaaaaagga agagt 105

<210> 14
<211> 87
<212> DNA
<213> Artificial Sequence

<220>
<223> rrnB T1 terminator

<400> 14
caaataaaac gaaaggctca gtcgaaagac tgggcctttc gttttatctg ttgtttgtcg 60

gtgaacgctc tcctgagtag gacaaat 87

<210> 15
<211> 28
<212> DNA
<213> Artificial Sequence

<220>
<223> rrnB T2 terminator

<400> 15
agaaggccat cctgacggat ggcctttt                                          28


<210> 16
<211> 589
<212> DNA
<213> Artificial Sequence


<220>
<223> pMB1 origin of replication

<400> 16
tttccatagg ctccgccccc ctgacgagca tcacaaaaat cgacgctcaa gtcagaggtg      60

gcgaaacccg acaggactat aaagatacca ggcgtttccc cctggaagct ccctcgtgcg     120

ctctcctgtt ccgaccctgc cgcttaccgg atacctgtcc gcctttctcc cttcgggaag     180

cgtggcgctt tctcatagct cacgctgtag gtatctcagt tcggtgtagg tcgttcgctc     240

caagctgggc tgtgtgcacg aaccccccgt tcagcccgac cgctgcgcct tatccggtaa     300

ctatcgtctt gagtccaacc cggtaagaca cgacttatcg ccactggcag cagccactgg     360

taacaggatt agcagagcga ggtatgtagg cggtgctaca gagttcttga agtggtggcc     420

taactacggc tacactagaa ggacagtatt tggtatctgc gctctgctga agccagttac     480

cttcggaaaa agagttggta gctcttgatc cggcaaacaa accaccgctg gtagcggtgg     540

tttttttgtt tgcaagcagc agattacgcg cagaaaaaaa ggatctcaa                 589


<210> 17
<211> 6088
<212> DNA
<213> Artificial Sequence


<220>
<223> KTXHIS-pelbLC-pelbHC

<400> 17
atctttctgc gaattgagat gacgccactg gctgggcgtc atcccggttt cccgggtaaa      60

caccaccgaa aaatagttac tatcttcaaa gccacattcg gtcgaaatat cactgattaa     120

caggcggcta tgctggagaa gatattgcgc atgacacact ctgacctgtc gcagatattg     180

attgatggtc attccagtct gctggcgaaa ttgctgacgc aaaacgcgct cactgcacga     240

tgcctcatca caaaatttat ccagcgcaaa gggacttttc aggctagccg ccagccgggt     300

aatcagctta tccagcaacg tttcgctgga tgttggcggc aacgaatcac tggtgtaacg     360

atggcgattc agcaacatca ccaactgccc gaacagcaac tcagccattt cgttagcaaa     420

cggcacatgc tgactacttt catgctcaag ctgaccaata acctgccgcg cctgcgccat     480

ccccatgcta cctaagcgcc agtgtggttg ccctgcgctg gcgttaaatc ccggaatcgc 540

cccctgccag tcaagattca gcttcagacg ctccgggcaa taaataatat tctgcaaaac 600

cagatcgtta acggaagcgt aggagtgttt atcatcagca tgaatgtaaa agagatcgcc 660

acgggtaatg cgataagggc gatcgttgag tacatgcagg ccattaccgc gccagacaat 720

caccagctca caaaaatcat gtgtatgttc agcaaagaca tcttgcggat aacggtcagc 780

cacagcgact gcctgctggt cgctggcaaa aaaatcatct ttgagaagtt ttaactgatg 840

cgccaccgtg gctacctcgg ccagagaacg aagttgatta ttcgcaatat ggcgtacaaa 900

tacgttgaga agattcgcgt tattgcagaa agccatcccg tccctggcga atatcacgcg 960

gtgaccagtt aaactctcgg cgaaaaagcg tcgaaaagtg gttactgtcg ctgaatccac 1020

agcgataggc gatgtcagta acgctggcct cgctgtggcg tagcagatgt cgggctttca 1080

tcagtcgcag gcggttcagg tatcgctgag gcgtcagtcc cgtttgctgc ttaagctgcc 1140

gatgtagcgt acgcagtgaa agagaaaatt gatccgccac ggcatcccaa ttcacctcat 1200

cggcaaaatg gtcctccagc caggccagaa gcaagttgag acgtgatgcg ctgttttcca 1260

ggttctcctg caaactgctt ttacgcagca agagcagtaa ttgcataaac aagatctcgc 1320

gactggcggt cgagggtaaa tcattttccc cttcctgctg ttccatctgt gcaaccagct 1380

gtcgcacctg ctgcaatacg ctgtggttaa cgcgccagtg agacggatac tgcccatcca 1440

gctcttgtgg cagcaactga ttcagcccgg cgagaaactg aaatcgatcc ggcgagcgat 1500

acagcacatt ggtcagacac agattatcgg tatgttcata cagatgccga tcatgatcgc 1560

gtacgaaaca gaccgtgcca ccggtgatgg tatagggctg cccattaaac acatgaatac 1620

ccgtgccatg ttcgacaatc acaatttcat gaaaatcatg atgatgttca ggaaaatccg 1680

cctgcgggag ccggggttct atcgccacgg acgcgttacc agacggaaaa aaatccacac 1740

tatgtaatac ggtcatactg gcctcctgat gtcgtcaaca cggcgaaata gtaatcacga 1800

ggtcaggttc ttaccttaaa ttttcgacgg aaaaccacgt aaaaaacgtc gatttttcaa 1860

gatacagcgt gaattttcag gaaatgcggt gagcatcaca tcaccacaat tcagcaaatt 1920

gtgaacatca tcacgttcat ctttccctgg ttgccaatgg cccattttct tgtcagtaac 1980

gagaaggtcg cgaatccagg cgctttttag actggtcgta atgaaattca ggaggaattc 2040

ctaatgaaat atctgctgcc gaccgcagca gcgggtctgc tgctgctggc agcacagcct 2100

gcaatggcag atattcagct gacccagagc ccgagcagcc tgagcgcaag cgttggtgat 2160

cgtgttacca ttacctgtag cgcaagccag gatattagca attatctgaa ttggtatcag 2220

cagaaaccgg gtaaagcacc gaaagtgctg atctatttta ccagcagcct gcatagcggt 2280

gttccgagcc gttttagcgg tagcggtagt ggcaccgatt ttaccctgac cattagcagc 2340

ctgcagccgg aagattttgc aacctattat tgtcagcagt atagcaccgt tccgtggacc 2400

tttggtcagg gcaccaaagt tgaaattaaa cgtaccgttg cagcaccgag cgtttttatc 2460

tttccgccta gtgatgaaca gctgaaaagc ggcaccgcaa gcgttgtttg tctgctgaat 2520

aacttttatc cgcgtgaagc aaaagttcag tggaaagttg ataatgcact gcagagcggt 2580

aatagccaag aaagcgttac cgaacaggat agcaaagata gcacctatag cctgagcagc 2640

accctgaccc tgagcaaagc agattatgaa aaacacaaag tgtatgcctg cgaagttacc 2700

catcagggtc tgagcagtcc ggttaccaaa agttttaatc gtggtgaatg ctaataatct 2760

agaactgtta tcgatatcag gaggattagc atatgaaata tctgctgccg accgcagcag 2820

cgggtctgct gctgctggca gcacagcctg caatggcaga agttcagctg gttgaaagcg 2880

gtggtggtct ggttcagcct ggtggtagcc tgcgtctgag ctgtgcagca agcggttatg 2940

attttaccca ttatggtatg aattgggttc gtcaggcacc gggtaaaggt ctggaatggg 3000

ttggttggat taatacctat accggtgaac cgacctatgc agcagatttt aaacgtcgtt 3060

ttacctttag cctggatacc agcaaaagca ccgcatatct gcagatgaat agcctgcgtg 3120

cagaggatac cgcagtgtat tattgtgcaa aatatccgta ttattacggc accagccatt 3180

ggtatttcga tgtttggggt cagggcaccc tggttaccgt tagcagcgca agcaccaaag 3240

gtccgagcgt ttttccgctg gcaccgagca gcaaaagtac cagcggtggc accgcagcac 3300

tgggttgtct ggttaaagat tattttccgg aaccggttac cgtgagctgg aatagcggtg 3360

cactgaccag cggtgttcat acctttccgg cagttctgca gagcagcggt ctgtatagcc 3420

tgagcagcgt tgttaccgtt ccgagcagca gcctgggcac ccagacctat atttgtaatg 3480

ttaatcataa accgagcaat accaaagtgg ataaaaaagt ggaaccgaaa agctgcgata 3540

aaacccatct gtaataaaag cttggctgtt ttggcggatg agagaagatt ttcagcctga 3600

tacagattaa atcagaacgc agaagcggtc tgataaaaca gaatttgcct ggcggcagta 3660

gcgcggtggt cccacctgac cccatgccga actcagaagt gaaacgccgt agcgccgatg 3720

gtagtgtggg gtctccccat gcgagagtag ggaactgcca ggcatcaaat aaaacgaaag 3780

gctcagtcga aagactgggc ctttcgtttt atctgttgtt tgtcggtgaa cgctctcctg 3840

agtaggacaa atccgccggg agcggatttg aacgttgcga agcaacggcc cggagggtgg 3900

cgggcaggac gcccgccata aactgccagg catcaaatta agcagaaggc catcctgacg 3960

gatggccttt ttgcgtttct acaaactctt ttgtttattt ttctaaatac attcaaatat 4020

gtatccgctc atgagactag gcttccgcgc cctcatccga aagggcgtat tcatatatgc 4080

ggtgtgaaat accgcacaga tgcgtaagga gaaaataccg catcaggcgc tcttccgctt 4140

cctcgctcac tgactcgctg cgctcggtcg ttcggctgcg gcgagcggta tcagctcact 4200

caaaggcggt aatacggtta tccacagaat caggggataa cgcaggaaag aacatgtgag 4260

caaaaggcca gcaaaaggcc aggaaccgta aaaaggccgc gttgctggcg tttttccata 4320

ggctccgccc ccctgacgag catcacaaaa atcgacgctc aagtcagagg tggcgaaacc 4380

cgacaggact ataaagatac caggcgtttc cccctggaag ctccctcgtg cgctctcctg 4440

ttccgaccct gccgcttacc ggatacctgt ccgcctttct cccttcggga agcgtggcgc 4500

tttctcatag ctcacgctgt aggtatctca gttcggtgta ggtcgttcgc tccaagctgg 4560

gctgtgtgca cgaacccccc gttcagcccg accgctgcgc cttatccggt aactatcgtc 4620

ttgagtccaa cccggtaaga cacgacttat cgccactggc agcagccact ggtaacagga 4680

ttagcagagc gaggtatgta ggcggtgcta cagagttctt gaagtggtgg cctaactacg 4740

gctacactag aaggacagta tttggtatct gcgctctgct gaagccagtt accttcggaa 4800

aaagagttgg tagctcttga tccggcaaac aaaccaccgc tggtagcggt ggtttttttg 4860

tttgcaagca gcagattacg cgcagaaaaa aaggatctca agaagatcct ttgatctttt 4920

ctacggggtc tgacgctcag tggaacgaaa actcacgtta agggattttg gtcatgagat 4980

tatcaaaaag gatcttcacc tagatccttt taaattaaaa atgaagtttt aaatcaatct 5040

aaagtatata tgagtaaact tggtctgaca gttagaaaaa ctcatcgagc atcaaatgaa 5100

actgcaattt attcatatca ggattatcaa taccatattt ttgaaaaagc cgtttctgta 5160

atgaaggaga aaactcaccg aggcagttcc ataggatggc aagatcctgg tatcggtctg 5220

cgattccgac tcgtccaaca tcaatacaac ctattaattt cccctcgtca aaaataaggt 5280

tatcaagtga gaaatcacca tgagtgacga ctgaatccgg tgagaatggc aaaagtttat 5340

gcatttcttt ccagacttgt tcaacaggcc agccattacg ctcgtcatca aaatcactcg 5400

catcaaccaa accgttattc attcgtgatt gcgcctgagc gagacgaaat acgcgatcgc 5460

tgttaaaagg acaattacaa acaggaatcg aatgcaaccg gcgcaggaac actgccagcg 5520

catcaacaat attttcacct gaatcaggat attcttctaa tacctggaat gctgttttcc 5580

cggggatcgc agtggtgagt aaccatgcat catcaggagt acggataaaa tgcttgatgg 5640

tcggaagagg cataaattcc gtcagccagt ttagtctgac catctcatct gtaacatcat 5700

tggcaacgct acctttgcca tgtttcagaa acaactctgg cgcatcgggc ttcccataca 5760

atcgatagat tgtcgcacct gattgcccga cattatcgcg agcccattta tacccatata 5820

aatcagcatc catgttggaa tttaatcgcg gcctagagca agacgtttcc cgttgaatat 5880

ggctcatact cttccttttt caatattatt gaagcattta tcagggttat tgtctcatga 5940

gcggatacat atttgaatgt atttagaaaa ataaacaaat aggggttccg cgcacatttc 6000

cccgaaaagt gccacctgac gtctaagaaa ccattattat catgacatta acctataaaa 6060

ataggcgtat cacgaggccc tttcgtct 6088

<210> 18
<211> 22
<212> PRT
<213> Erwinia

<220>
<223> PelB signal peptide - peptide

<400> 18
Met Lys Tyr Leu Leu Pro Thr Ala Ala Ala Gly Leu Leu Leu Leu Ala
1               5                   10                  15
Ala Gln Pro Ala Met Ala
            20

<210> 19
<211> 32
<212> DNA
<213> Artificial Sequence

<220>
<223> SBamHI1680

<400> 19
gtacgctagg atcctgtggc agcaactgat tc                                32

<210> 20
<211> 32
<212> DNA
<213> Artificial Sequence

<220>
<223> BSalI1681

<400> 20
gtagataggt cgacagcgat cgtcattggg at                                32